# EUROPEAN PATENT APPLICATION

(11) **EP 4 056 038 A1**
(43) Date of publication of application: **14.09.2022**
(21) Application number: 21161748.5
(22) Date of filing: 10.03.2021
(51) Int. Cl.: A01N 25/28, A01N 43/90, A01N 43/10, A01P 13/00

(54) **MICROPARTICLES CONTAINING ACTIVE SUBSTANCES**

(71) Applicant: BASF SE, 67056 Ludwigshafen am Rhein (DE)
(72) Inventor: TARANTA, Claude, 67117 Limburgerhof (DE); OSCHMANN, Bernd Dieter, 67056 Ludwigshafen (DE); INCERA GARRIDO, Gerardo, 200137 Shanghai (CN); BEZIAU, Antoine Maxine Charles Joseph, 67056 Ludwigshafen (DE); BORK, Thomas, 67117 Limburgerhof (DE); VOLLAND, Thorsten, 67117 Limburgerhof (DE); HENNING, Urch, 67117 Limburgerhof (DE); KRAEMER, Gerd, 67117 Limburgerhof (DE); PICARD, Laurent, 67117 Limburgerhof (DE); KIENLE, Marcel Patrik, 67117 Limburgerhof (DE); KRAUS, Helmut, L4W 0B6 Mississauga (CA); MARCHIORO, Carla, 40121 Bologna (IT); BASSETTI, Lucio, 40121 Bologna (IT); GOBBI, Carlotta, 40121 Bologna (IT); BORZATTA, Valerio, 40121 Bologna (IT)
(74) Representative: BASF IP Association

(57) **Abstract**

Microparticle, wherein said microparticle contains one or more active substance, said one or more active substance being water immiscible, and being liquid (at 21 °C) pesticide or dissolved in a non-aqueous solvent S that is immiscible with water, and
wherein said microparticle contains
i) at least one phospholipid PL,
ii) optionally at least one sterol ST.

## Description

The present invention is directed to microparticles, wherein said microparticles contain one or more active substance, said one or more active substance being water immiscible, wherein said one or more active substance is liquid (at 21 °C) or dissolved in a non-aqueous solvent S that is immiscible with water, and
wherein said microparticles contain
i) at least one phospholipid PL, and
ii) optionally at least one sterol ST.

It is further directed to processes for making such microparticles and for their uses and to formulations and uses of such microparticles.

The encapsulation of active substances has been known for a long time. It offers several advantages over non-encapsulated formulations. For example, it is possible to control the release profile of the active substance in the formulation.

Known encapsulation technologies involve for example the formation of polymer shells or polymer particles around an encapsulated active substance. Polymers often used for such encapsulation include acrylic polymers, polyurea or polyurethane polymers or aminoplast polymers.

It is a drawback of the abovementioned encapsulation technologies that the polymers are not easily biodegradable and may lead to the formation of small polymer particles that may persist over extended periods of time. Such persistent polymer particles are sometime also referred to as microplastics.

Is was therefore the objective of the present invention to provide microparticles containing active substances that have excellent release profiles and form stable formulations, yet at the same time can degrade easily under ambient conditions and do not form microplastics.

This objective was achieved by microparticles, wherein said microparticles contain one or more active substance, said one or more active substance being water immiscible, wherein said one or more active substance is liquid (at 21 °C) or dissolved in a non-aqueous solvent S that is immiscible with water, and
wherein said microparticles contain
i) at least one phospholipid PL, and
ii) optionally at least one sterol ST.

In one embodiment, this objective was achieved by microparticles, wherein said microparticles contain one or more active substance, said one or more active substance being water immiscible, wherein said one or more active substance is liquid (at 21 °C) or dissolved in a non-aqueous solvent S that is immiscible with water, and
wherein said microparticles contain
i) at least one phospholipid PL, and
ii) at least one sterol ST.

In one embodiment, this objective was achieved by microparticles, said microparticles being microcapsules having a shell and a core or being microspheres, wherein said microspheres or the core of said microcapsules contain one or more active substance, said one or more active substance being water immiscible, wherein said one or more active substance is liquid (at 21 °C) or dissolved in a non-aqueous solvent S that is immiscible with water, and
wherein said microspheres or the shell of said microcapsules contain
i) at least one phospholipid PL, and
ii) optionally at least one sterol ST.

In one preferred embodiment, this objective was achieved by microparticles, said microparticles being microcapsules having a shell and a core or being microspheres, wherein said microspheres or the core of said microcapsules contain one or more active substance, said one or more active substance being water immiscible, wherein said one or more active substance is liquid (at 21 °C) or dissolved in a non-aqueous solvent S that is immiscible with water, and
wherein said microspheres or the shell of said microcapsules contain
i) at least one phospholipid PL, and
ii) at least one sterol ST.

Microparticles of the invention are typically microcapsules having a shell and a core or are microspheres.

The term microsphere as used herein denotes a particulate structure characterized by the absence of an outer shell, an outer membrane, or any distinct outer layer, having an average particle size as described below that contains one or more active substance distributed in a matrix material. According to the invention, said matrix material comprises at least one phospholipid PL and optionally at least one sterol ST as the main components by weight. Microspheres according to the invention are typically liquid or semiliquid ("jellylike"). Microspheres of the invention are typically spherical.

The active substance can be partly dissolved in the matrix material and partly present in a separate phase as droplets that are distributed in the matrix.

The microparticles of the invention contain in the matrix of the microsphere or in the core of the microcapsule one or more active substances. An active substance as used herein shall mean any chemical compound or mixture of compounds that is used to achieve a certain effect on a target upon its release.

According to the invention, active substances contained in the microparticles of the invention are immiscible with water.

"immiscible with water" in this context shall mean that such active substances have a solubility in water of less than 10 g/l at 21 °C, preferably less than 1 g/l at 21 °C. In one embodiment, water immiscible active substances have a solubility in water of less than 0.1 g/l at 21 °C.

In one embodiment, active substances are selected from pesticides, plant health agents, repellants, biocides, phase-change materials, pharmaceuticals, cosmetic ingredients (like fragrances, perfumes, vitamins, essential oils, plant extracts), nutrients, food additives (like vegetable oils, marine oils, vitamins, aromas, antioxidants, essential oils, plant extracts), pheromones, catalysts.

Preferred active substances are selected from pesticides, pharmaceuticals, cosmetic ingredients (like fragrances, perfumes, vitamins, essential oils, plant extracts), nutrients, food additives (like vegetable oils, marine oils, vitamins, aromas, antioxidants, essential oils, plant extracts), pheromones and catalysts.

In one embodiment, active substances are selected from pesticides, cosmetic ingredients (like fragrances, perfumes, vitamins, essential oils, plant extracts), nutrients, food additives (like vegetable oils, marine oils, vitamins, aromas, antioxidants, essential oils, plant extracts), pheromones and catalysts.

In one embodiment, active substances are selected from pesticides.
In one embodiment, active substances are selected from active substances for personal care.
In one embodiment, active substances are selected from cosmetic ingredients (like fragrances, perfumes, vitamins, essential oils, plant extracts).
In one embodiment, active substances are selected nutrients.
In one embodiment, active substances are selected from food additives (like vegetable oils, marine oils, vitamins, aromas, antioxidants, essential oils, plant extracts).
In one embodiment, active substances are selected from pheromones.
In one embodiment, active substances are selected from catalysts.
In one embodiment, active substances are selected from insect repellants.
In one embodiment, active substances are selected from biocides.

In one embodiment, active substances are nutrients that are used in the food and animal nutrition sector, such as lipophilic vitamins, such as, for example, tocopherol, vitamin A and derivatives thereof, vitamin D and derivatives thereof, vitamin K and derivatives thereof, vitamin E, vitamin F and derivatives thereof, or saturated and unsaturated fatty acids, and also derivatives and compounds thereof, natural and synthetic flavorings, aroma substances and fragrances and lipophilic dyes, such as, for example, retinoids, flavonoids or carotenoids.

In one embodiment, active substances are pharmaceuticals such as anesthetics and narcotics, anticholinergics, antidepressants, psychostimulants, and neuroleptics, antiepileptics, antimycotics, antiphlogistics, bronchodilators, cardiovascular medicaments, cytostatics, hyperemics, antilipemics, spasmolytics, testosterone derivatives, tranquilizers or virustatics.

In one embodiment, active substances are food additives like vegetable oils, marine oils, vitamins, aromas, antioxidants, essential oils, plant extracts.
One preferred active substance is vitamin A.
One preferred active substance is vitamin E.

One preferred type active substance are edible oils (e.g. vegetable or marine oils) containing unsaturated fatty acids, such as omega-3 fatty acids or mire highly unsaturated fatty acids such as docosahexaenoic acid or eicosapentaenoic acid.

In one embodiment, active substances are substances used in the field of personal care (e.g. cosmetics), such as perfume oils, organic UV filters, dyes, or care substances, such as panthenol.

In one embodiment, active substances are preferred dyes which can be used as active substances in the capsules according to the invention are natural or synthetic dyes which are approved in the field of nutrition or of cosmetics, as are described, for example, in WO 2005/009604 A1 on page 9, lines 18 to 30.

In one embodiment, the active substance is an organic UV filter. Examples of such organic UV filters are the following commercially available UV filters:
PABA, Homosalate (HMS), Benzophenone-3 (BENZ-3), Butyl Methoxydibenzoylmethane (BMDBM), Octocrylene (OC), Polyacrylamidomethyl Benzylidene Camphor, Ethylhexyl Methoxycinnamate (EMC, OMC), Isoamyl p-Methoxycinnamate (IMC), Ethylhexyl Triazone (OT, ET), Drometrizole Trisiloxane, Diethylhexyl Butamido Triazone (DBT), 4-Methylbenzylidene Camphor (MBC), 3-Benzylidene Camphor (BC), Ethylhexyl Salicylate (OS, ES), Ethylhexyl Dimethyl PABA (OD-PABA, ED-PABA), Benzophenone-4 (BENZ-4), Methylene Bis-Benzotriazolyl Tetramethylbutylphenol (Bisoctyltriazol, BOT), Bis-Ethylhexyloxyphenol Methoxyphenyl Triazine (AT), Polysilicone 15 or Diethylamino Hydroxybenzoyl Hexyl Benzoate, and mixtures of these UV filters. Further UV filters can likewise be used: 2,4,6-Tris (biphenyl )-1,3,5-triazine (TBT), Methanone 1,1'-(1,4-piperazinediyl)bis[1-[2-[4-(diethylamino)-2-hydroxybenzoyl]phenyl]] (CAS number 919803-06-8), 1,1-di(carboxy-(2',2'-dimethylpropyl))-4,4-diphenylbutadiene, merocyanine derivatives or benzylidene malonate UVB filters, and also mixtures of these UV filters with one another or with the UV filters.

Particular preference is given to UV filters selected from Octocrylene, Ethylhexyl Methoxycinnamate, Ethylhexyl Triazone, Diethylamino Hydroxybenzoyl Hexyl Benzoate, Methylene Bis-Benzotriazolyl Tetramethylbutylphenol or Bis-Ethylhexyloxyphenol Methoxyphenyl Triazine, and mixtures of these UV filters.

Suitable pheromones as active substances include the following:

| Name | CAS # |
|---|---|
| Bicyclo[3.1.1]hept-3-en-2-one, 4,6,6-trimethyl-, (1S)- | 1196-01-6 |
| Bicyclo[3.1.1]hept-3-en-2-ol, 4,6,6-trimethyl-, [1S-(1a,2b,5a)]- | 18881-04-4 |
| 2,6-Octadienal, 3,7-dimethyl- | 5392-40-5 |
| MIXTURE/ Acetaldehyde, (3,3-dimethylcyclohexylidene)-, (2Z)-and Acetaldehyde, (3,3-dimethylcyclohexylidene)-, (2E)-2,7-Octadien-4-ol, 2-methyl-6-methylene- | 14434-41-4 |
| Ethanol, 2-(3,3-dimethylcyclohexylidene)-, (2E)- | 30346-27-1 |
| Cyclobutaneethanol, 1-methyl-2-(1-methylethenyl)-, cis- | 30820-22-5 |
| Ethanol, 2-(3,3-dimethylcyclohexylidene)-, (2Z)- | 26532-23-0 |
| 7-Octen-4-ol, 2-methyl-6-methylene- | 30894-96-4 |
| 5-Nonanone, 4-methyl- | 35900-26-6 |
| 5-Decen-1-ol, (5E)- | 56578-18-8 |
| 5-Decen-1-ol, (5Z)- | 51652-47-2 |
| 5-Nonanol, 4-methyl- | 154170-44-2 |
| 5-Nonanol, 4-methyl- | 154170-44-2 |
| 2,4,6-Decatrienoic acid, methyl ester (2E,4E,6Z)- | 51544-64-0 |
| 2,4-Decadienoic acid, methyl ester, (2E,4Z)- | 4493-42-9 |
| MIXTURE/ Nonan-3-one, 4-6-dimethyl-7-hydroxy-: (4R,6S,7S)-(.+-.)-; (4R,6R,7R)-(.+-.)- | [99945-27-4] and [92999-14-9] |
| 8,10-Dodecadien-1-ol, (8E,10E)- | 33956-49-9 |
| 5-Decen-1-ol, acetate, (5E)- | 38421-90-8 |
| 3-Decen-1-ol, acetate, (3Z)- | 81634-99-3 |
| 5-Decen-1-ol, acetate, (5Z)- | 37446-07-5 |
| 7-Decen-1-ol, acetate, (7Z)- | 13857-03-9 |
| 8-Dodecen-1-ol, (8Z)- | 40642-40-8 |
| 8-Dodecen-1-ol, (8Z)- | 40642-40-8 |
| 9-Dodecen-1-ol, (9Z)- | 35148-18-6 |
| 8,10-Dodecadien-1-ol, acetate, (8E,10E)- | 53880-51-6 |
| 7,9-Dodecadien-1-ol, acetate, (7E,9Z)- | 54364-62-4 |
| MIXTURE/ 11-Tetradecenal, (11E)- and 11-Tetradecenal, (11Z)- | [35746-21-5] and [35237 -64-0] |
| 11-Tetradecenal, (11Z)- | 35237 -64-0 |
| 9-Tetradecenal, (9Z)- | 53939-27-8 |
| 9,12-Tetradecadien-1-ol, (9Z,12E)- | 51937-00-9 |
| 7-Tetradecen-2-one, (7Z)- | 146955-45-5 |
| 11-Dodecen-1-ol, acetate | 35153-10-7 |
| 7-Dodecen-1-ol, acetate, (7E)- | 16695-41-3 |
| 8-Dodecen-1-ol, acetate, (8E)- | 38363-29-0 |
| 9-Dodecen-1-ol, acetate, (9E)- | 35148-19-7 |
| MIXTURE/ 8-Dodecen-1-ol, 1-acetate, (8E)- and 8-Dodecen-1-ol, 1-acetate, (8Z)- | [38363-29-0] and [28079-04-1] |
| 5-Dodecen-1-ol, acetate, (5Z)- | 16676-96-3 |
| 7-Dodecen-1-ol, acetate, (7Z)- | 14959-86-5 |
| 8-Dodecen-1-ol, acetate, (8Z)- | 28079-04-1 |
| 9-Dodecen-1-ol, acetate, (9Z)- | 16974-11-1 |
| 11-Tetradecen-1-ol, (11 E)- | 35153-18-5 |
| 11-Tetradecen-1-ol, (11Z)- | 34010-15-6 |
| 9-Tetradecen-1-ol, (9Z)- | 35153-15-2 |
| 1,6,10-Dodecatriene, 7,11-dimethyl-3-methylene-, (6E)- | 18794-84-8 |
| MIXTURE/ 4-Tridecen-1-ol, acetate, (4E)- and 4-Tridecen-1-ol, acetate, (4Z)- | [72269-48-8] and [65954-19-0] |
| 4-Tridecen-1-ol, acetate, (4Z)- | 35954-19-0 |
| 11,13-Hexadecadienal, (11Z,13Z)- | 71317-73-2 |
| 9,11-Tetradecadien-1-ol, acetate, (9E,11E)- | 54664-98-1 |
| 9,12-Tetradecadien-1-ol, acetate, (9Z,12E)- | 30507-70-1 |
| 9,11-Tetradecadien-1-ol, acetate, (9Z,11E)- | 50767-79-8 |
| 11-Hexadecenal, (11Z)- | 53939-28-9 |
| 9-Hexadecenal, (9Z)- | 56219-04-6 |
| 9-Hexadecenal, (9Z)- | 56219-04-6 |
| 11-Tetradecen-1-ol, acetate, (11Z)- | 20711-10-8 |
| 11-Tetradecen-1-ol, acetate, (11E)- | 33189-72-9 |
| 9-Tetradecen-1-ol, acetate, (9E)- | 23192-82-7 |
| 7-Tetradecen-1-ol, acetate, (7Z)- | 16974-10-0 |
| 8-Tetradecen-1-ol, acetate, (8Z)- | 35835-80-4 |
| 9-Tetradecen-1-ol, acetate, (9Z)- | 16725-53-4 |
| 11-Hexadecen-1-ol, (11E)- | 31301-56-2 |
| 11-Hexadecen-1-ol, (11Z)- | 56683-54-6 |
| 8-Hexadecenal, 14-methyl-, (8Z)- | 30609-53-2 |
| 6-acetoxy-5-Hexadecanolide | 81792-36-1 |
| 13-Octadecenal, (13Z)- | 58594-45-9 |
| 11-Hexadecen-1-ol, acetate, (11Z)- | 34010-21-4 |
| 11-Hexadecen-1-ol, acetate, (11E)- | 56218-72-5 |
| MIXTURE/ 2,13-Octadecadien-1-ol, acetate, (2E,13Z)- [and 3,13-Octadecadien-1-ol, acetate, (3E,13Z)- | MIXTURE/ [86252-35-5] and [53120-26-6] |
| 7-Eicosen-11-one, (7Z)- | 33408-44-6 |
| 13-Octadecen-1-ol, acetate, (13Z)- | 30037-58-3 |
| 6-Heneicosen-11-one, (6Z)- | 54844-65-4 |
| 9-Tricosene, (9Z)- | 27519-02-4 |
| 2-Cyclohexen-1-one, 3-methyl- | 1193-18-6 |
| 1-Octen-3-ol | 3391-86-4 |
| 1-Octen-3-ol, (3R)- | 3687-48-7 |
| MIXTURE/: 8-Dodecen-1-ol, acetate, (8Z)-; 8-Dodecen-1-ol, acetate, (8E)-; 8-Dodecen-1-ol, (8Z)- | MIXTURE |
| MIXTURE/ 5-Decen-1-ol, acetate, (5E)- and 5-Decen-1-ol, (5E)- | MIXTURE/ [38421-90-8] and [56578-18-8] |
| MIXTURE/ 11-Tetradecen-1-ol, acetate, (11E)- and 9,11-Tetradecadien-1-ol, acetate, (9E,11E) | MIXTURE/ [33189-72-9] and [54664-98-1] |
| MIXTURE/ cis-2-Isopropenyl-1-methylcyclobutaneethanol, (Z)-2-(3,3-Dimethyl)-cyclohexylideneethanol, (Z)-(3, 3-Dimethyl)-cyclohexylideneacetaldehyde, (E)-(3,3-Dimethyl)-cyclohexylideneacetaldehyde | MIXTURE[30820-22-5], [26532-23-0],[26532-24-1],[26532-25-2] |
| Mixture of (Z)-9-hexadecenal, (Z)-11-hexadecenal and (Z)-13-octadecenal | |

In one preferred embodiment, said active substance is selected from the following list
(1S)-4,6,6-trimethyl bicyclo[3.1.1]hept-3-en-2-one; 3,7-dimethyl-bicyclo[3.1.1]hept-3-en-2-ol; 4,6,6-trimethyl-, [1S-(1a,2b,5 a)]-2,6-octadienal; (3,3-dimethylcyclohexylidene)-acetaldehyde; mixture of (2Z) (3,3-dimethylcyclohexylidene)-acetaldehyde and (2E) (3,3-dimethylcyclohexylidene)-acetaldehyde; 2-methyl-6-methylene-2,7-octadien-4-ol; (2E) 2-(3,3-dimethylcyclohexylidene)-ethanol; cis-1-methyl-2-(1-methylethenyl)-cyclobutaneethanol;
(2Z)- 2-(3,3-dimethylcyclohexylidene)-ethanol; 2-methyl-6-methylene-7-Octen-4-ol;
4-methyl-5-Nonanone; (5E)-5-Decen-1-ol; (5Z)- 5-Decen-1-ol; 4-methyl-5-Nonanol;
(2E,4E,6Z)-2,4,6-Decatrienoic acid methyl ester; (2E,4Z)-2,4-Decadienoic acid methyl ester; 4,6-dimethyl-7-hydroxy-nonan-3-one; mixture of (4R,6S,7S)-(.+-.)- 4,6-dimethyl-7-hydroxynonan-3-one and (4R,6R,7R)-(.+-.)- 4,6-dimethyl-7-hydroxy-nonan-3-one; (8E,10E)- 8,10-Dodecadien-1-ol; (5E)- 5-Decen-1-ol, acetate; (3Z)- 3-Decen-1-ol, acetate; (5Z)- 5-Decen-1-olacetate; (7Z)- 7-Decen-1-ol, acetate; (8Z)- 8-Dodecen-1-ol; (9Z)- 9-Dodecen-1-ol;(8E,10E)-8,10-Dodecadien-1-ol acetate; (7E,9Z)-7,9-Dodecadien-1-ol acetate;
11-tetradecenal; Mixture of (11E)- 11-Tetradecenal, and (11Z)-11-Tetradecenal;
(11Z)- 11-Tetradecenal; (9Z)- 9-Tetradecenal;(9Z,12E)-9,12-Tetradecadien-1-ol; (7Z)- 7-Tetradecen-2-one; 11-Dodecen-1-ol acetate; (7E)-7-Dodecen-1-ol acetate; (8E)- 8-Dodecen-1-ol acetate; (9E)-9-Dodecen-1-ol acetate; 8-Dodecen-1-ol -1-acetate; Mixture of (8E)- 8-Dodecen-1-ol -1-acetate and (8Z)-8-Dodecen-1-ol -1-acetate; (5Z)- 5-Dodecen-1-ol acetate; (7Z)-7-Dodecen-1-ol acetate; (8Z)- 8-Dodecen-1-ol acetate; (9Z)- 9-Dodecen-1-ol acetate (11E)-11-Tetradecen-1-ol; (11Z)- 11-Tetradecen-1-ol; (6E)-7,11-dimethyl-3-methylene-1,6,10-Dodecatriene; 4-tridecen-1-ol acetate; Mixture of (4E)- 4-tridecen-1-ol acetate and (4Z)- 4-tridecen-1-ol acetate; (4Z)- 4-Tridecen-1-ol acetate; (11Z,13Z)-11,13-Hexadecadienal; (9E,11E)-9,11-Tetradecadien-1-ol acetate; (9Z,12E)-9,12-Tetradecadien-1-ol acetate; (9Z,11E)-9,11-Tetradecadien-1-ol acetate; (11Z)-11-Hexadecenal; (9Z)-9-Hexadecenal; (11Z)-11-Tetradecen-1-ol acetate; (11E)-11-Tetradecen-1-ol acetate; (9E)-9-Tetradecen-1-ol acetate; (7Z)-7-Tetradecen-1-ol acetate; (8Z)-8-Tetradecen-1-ol acetate; (9Z)-9-Tetradecen-1-ol acetate; (11E)-11-Hexadecen-1-ol; (11Z)-11-Hexadecen-1-ol; (8Z)- 14-methyl-8-Hexadecenal; 6-acetoxy-5-Hexadecanolide; (13Z)- 13-Octadecenal; (11Z)-11-Hexadecen-1-ol acetate; (11E); 11-Hexadecen-1-ol acetate; 2,13-Octadecadien-1-ol acetate; Mixture of (2E,13Z)- 2,13-Octadecadien-1-ol acetate and (3E,13Z)- 2,13-Octadecadien-1-ol acetate; (7Z)-7-Eicosen-11-one; (13Z)-13-Octadecen-1-ol acetate; (6Z)-6-Heneicosen-11-one; (9Z)-9-Tricosene;
3-methyl-2-Cyclohexen-1-one; 1-Octen-3-ol; (3R)-1-Octen-3-ol; Mixture of 8-Dodecen-1-ol acetate and -(8Z)-Dodecen-1-ol; Mixture of (8Z)-8-Dodecen-1-ol acetate, (8E)-8-Dodecen-1-ol acetate and (8Z)-8-Dodecen-1-ol; 5-Decen-1-ol acetate; Mixture of (5E)-5-Decen-1-ol acetate and, and (5E)-5-Decen-1-ol; Mixture of (11E)- 11-Tetradecen-1-ol acetate, and (9E,11E)- 9,11-Tetradecadien-1-ol acetate; Mixture of Compounds with the CAS numbers [30820-22-5],[26532-23-0],[26532-24-1] and[26532-25-2]; Mixture of (Z)-9-hexadecenal, (Z)-11-hexadecenal and (Z)-13-octadecenal; L-carvone; citral; (*E,Z*)-7,9-dodecadien-1-yl acetate; ethyl formate; (*E,Z*)-2,4-ethyl decadienoate (pear ester); (*Z,Z,E*)-7,11,13-hexadecatrienal; heptyl butyrate; isopropyl myristate; lavanulyl senecioate; cis-jasmone; 2-methyl 1-butanol; methyl eugenol; methyl jasmonate; (*E,Z*)-2,13-octadecadien-1-ol; (*E,Z*)-2,13-octadecadien-1-ol acetate; (*E,Z*)-3,13-octa-decadien-1-ol; (*R*)-1-octen-3-ol; pentatermanone; (*E,Z,Z*)-3,8,11-tetradecatrienyl acetate; (*Z,E*)-9,12-tetradecadien-1-yl acetate; (*Z*)-7-tetradecen-2-one; (*Z*)-9-tetradecen-1-yl acetate; (*Z*)-11-tetradecenal; (*Z*)-11-tetradecen-1-ol; extract of *Chenopodium ambrosiodes;* Neem oil; Quillay extract or mixtures thereof.

When mixtures of different isomers or of different pheromones are used, these are typically used in a mass ratio of 1:100 to 100:1, preferably 1:10 to 10:1.
In case of ternary or higher mixtures such ration shall apply with respect to each combination of the mixing partners.

In one embodiment, said active substance is selected from L-carvone, citral, (*E*,*Z*)-7,9-dodecadien-1-yl acetate, ethyl formate, (*E,Z*)-2,4-ethyl decadienoate (pear ester), (*Z,Z,E*)-7,11,13-hexadecatrienal, heptyl butyrate, isopropyl myristate, lavanulyl senecioate, cis-jasmone, 2-methyl 1-butanol, methyl eugenol, methyl jasmonate, (*E,Z*)-2,13-octadecadien-1-ol, (*E*,*Z*)-2,13-octadecadien-1-ol acetate, (*E,Z*)-3,13-octadecadien-1-ol, (*R*)-1-octen-3-ol, pentatermanone, (*E,Z,Z*)-3,8,11-tetradecatrienyl acetate, (*Z,E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract or mixtures thereof.

In one embodiment, said active substance is selected from L-carvone, citral, (*E*,*Z*)-7,9-dodecadien-1-yl acetate, ethyl formate, (*E,Z*)-2,4-ethyl decadienoate (pear ester), (*Z,Z,E*)-7,11,13-hexadecatrienal, heptyl butyrate, isopropyl myristate, lavanulyl senecioate, cis-jasmone, 2-methyl 1-butanol, methyl eugenol, methyl jasmonate, (*E,Z*)-2,13-octadecadien-1-ol, (*E*,*Z*)-2,13-octadecadien-1-ol acetate, (*E,Z*)-3,13-octadecadien-1-ol, (*R*)-1-octen-3-ol, pentatermanone, (*E,Z,Z*)-3,8,11-tetradecatrienyl acetate, (*Z,E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-olor mixtures thereof.

In one preferred embodiment, said active substance is selected from (E,Z)-7,9-Dodecadienyl acetate; 11-Dodecenyl acetate; (E)-7-Dodecenyl acetate; (E)-11-Tetradecenyl acetate; (E)-9-Tetradecenyl acetate; (E)-11-Hexadecenyl acetate; (Z,Z)-7,11-Hexadecadienyl acetate; (E,Z)-4,7-Tridecadienyl acetate; (E,Z,Z)-4,7,10-Tridecatrienyl acetate; (Z,Z,E)-7,11,13-Hexadecatrienal; (Z,Z)-7,11-Hexadecadienal; (Z)-11-Hexadecenal; (Z)-11-Hexadecen-1-ol; (Z)-11-Hexadecenyl acetate; (Z)-7-Tetradecenal; (Z,E)-7,11-Hexadecadienyl acetate; (Z,E)-7,11-Hexadecadienal; (Z,E)-9,12-Tetradecadien-1-ol; (Z)-9-Tetradecen-1-ol;
(Z,E)-9,12-Tetradecadienyl acetate; (E)-9-Tetradecenyl acetate;
(Z)-7-Dodecenyl acetate; (E)-9-Tetradecenyl acetate; (Z,E)-9,11-Tetradecadienyl acetate; (E,Z)-10,12-Hexadecadienal; (E,E)-10,12-Hexadecadienal; (E)-7-Dodecenyl acetate; (E)-8-Dodecenyl acetate; (Z)-8-Dodecenyl acetate; (Z)-7-Dodecenyl acetate; (E,Z,Z)-3,8,11-Tetradecatrienyl acetate; (E,Z)-3,8-Tetradecadienyl acetate; (E,Z)-3,7,11-Trimethyl-2,6,10-dodecatrien-1-ol; (Z)-3,7,11-Trimethyl-1,6,10-dodecatrien-3-ol; (E)-3,7-Dimethyl-2,6-octadien-1-ol; 3,7-Dimethyl-6-octen-1-ol; 2-(3,3-dimethylcyclohexylidene)- (2E)- Ethanol; Cyclobutaneethanol, 1-methyl-2-(1-methylethenyl)-, cis-; Ethanol, 2-(3,3-dimethylcyclohexylidene)-, (2Z)-; cis-2-Isopropenyl-1-methylcyclobutaneethanol; 10-Methyltridecan-2-one; 8-Methyldecan-2-yl propionate;
Butyl butyrate; (E)-2-Butenyl butyrate; (Z,E)-4,4-(1,5-Dimethyl-4-heptenylidene)-1-methylcyclohexene; Ethyl 2-propenoate; 4-Hydroxy-3-methoxybenzaldehyde; (E)-2-Decenal; 1-Methyl-4-(1,5-dimethyl-(Z)-1,4-hexadienyl)-cyclohexene; (1S,2R,4S)-4-(1,5-Dimethyl-(Z)-1,4-hexadienyl)-1,2-epoxy-1-methylcyclohexane; (1R,2S,4S)-4-(1,5-Dimethyl-(Z)-1,4-hexadienyl)-1,2-epoxy-1-methylcyclohexane; Hexyl hexanoate; (E)-2-Hexenyl hexanoate; Octyl butyrate; 3-Methyl-6-isopropenyl-9-decenyl acetate; (Z)-3-Methyl-6-isopropenyl-3,9-decadienyl acetate;
(E)-7,11-Dimethyl-3-methylene-1,6,10-dodecatriene; (1S,2R,3S)-2-(1-Formylvinyl)-5-methylcyclopentanecarbaldehyde; (1R,4aS,7S,7aR)-Hexahydro-4,7-dimethylcyclopenta[c]pyran-1-ol; (4aS, 7S, 7aR)-Tetrahydro-4,7-dimethylcyclopenta[c]pyranone;
2-Phenylacetonitrile; (S)-5-Methyl-2-(prop-1-en-2-yl)-hex-4-enyl 3-methyl-2-butenoate; (S)-5-Methyl-2-(prop-1-en-2-yl)-hex-4-enyl 3-methylbutanoate; (S)-5-Methyl-2-(prop-1-en-2-yl)-hex-4-en-1-ol; (Z)-3,7-Dimethyl-2,7-octadienyl propionate; (E)-3,7-Dimethyl-2,7-octadienyl propionate; 3-Methylene-7-methyl-7-octenyl propionate or mixtures thereof.

In one preferred embodiment, said active substance is selected from (E,Z)-7,9-Dodecadienyl acetate; 11-Dodecenyl acetate; (E)-7-Dodecenyl acetate; (E)-11-Tetradecenyl acetate; (E)-9-Tetradecenyl acetate; (E)-11-Hexadecenyl acetate; (Z,Z)-7,11-Hexadecadienyl acetate; (E,Z)-4,7-Tridecadienyl acetate; (E,Z,Z)-4,7,10-Tridecatrienyl acetate; (Z,Z,E)-7,11,13; Hexadecatrienal; (Z,Z)-7,11-Hexadecadienal; (Z)-11-Hexadecenal; (Z)-11-Hexadecen-1-ol; (Z)-11-Hexadecenyl acetate; (Z)-7-Tetradecenal; (Z,E)-7,11-Hexadecadienyl acetate; (Z,E)-7,11-Hexadecadienal; (Z,E)-9,12-Tetradecadien-1-ol; (Z)-9-Tetradecen-1-ol; (Z,E)-9,12; Tetradecadienyl acetate; (E)-9-Tetradecenyl acetate; (Z)-7-Dodecenyl acetate; (E)-9-Tetradecenyl acetate; (Z,E)-9,11-Tetradecadienyl acetate; (E,Z)-10,12-Hexadecadienal; (E,E)-10,12-Hexadecadienal; (E)-7-Dodecenyl acetate; (E)-8-Dodecenyl acetate; (Z)-8-Dodecenyl acetate; (Z)-7-Dodecenyl acetate; (E,Z,Z)-3,8,11-Tetradecatrienyl acetate; (E,Z)-3,8-Tetradecadienyl acetate or mixtures thereof.

Preferred insect repellants as active substances are ethyl butylacetylaminopropionate, diethyltoluamide, picaridin, 2-undecanone.

In one embodiment, active substances are **pesticides** such as insecticides, fungicides, nematicides, rodenticides, molluscicides, growth regulators, herbicides or biocides.
In one embodiment, active substances are **pesticides** such as insecticides, fungicides, nematicides, rodenticides, molluscicides, growth regulators and herbicides.
Preferred pesticides are insecticides, fungicides and herbicides.

The term pesticide (or agrochemical active substance) refers to at least one active substance selected from the group of fungicides, insecticides, nematicides, herbicides, rodenticides, safeners and/or growth regulators. Preferred pesticides are fungicides, insecticides, rodenticides and herbicides. Mixtures of pesticides of two or more of the aforementioned classes can also be used. The person skilled in the art is familiar with such pesticides, which can be found, for example, in the Pesticide Manual, 14th ed. (2006), The British Crop Protection Council, London.

### Pesticides:

The U.S Environmental Protection Agency (EPA) defines a pesticide as "any substance or mixture of substances intended for preventing, destroying, repelling, or mitigating any pest". The skilled worker is familiar with such pesticides, which can be found, for example, in the Pesticide Manual, 16th Ed. (2013), The British Crop Protection Council, London. A pesticide may be a chemical substance or biological agent (such as a virus or bacteria) used against pests including insects, plant pathogens, weeds, mollusks, birds, mammals, fish, nematodes (roundworms) and microbes that compete with humans for food, destroy property, spread disease or are a nuisance. In the following examples, pesticides suitable for the agrochemical compositions according to the present invention are given.

Fungicides: Fungicides are chemical compounds used to prevent the spread of fungi in gardens and crops. Fungicides are also used to fight fungal infections. Fungicides can either be contact or systemic. A contact fungicide kills fungi when sprayed on its surface. A systemic fungicide has to be absorbed by the fungus before the fungus dies. Examples for suitable fungicides, according to the present invention, encompass the following compounds from the classes A) to L):
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-*N*-methoxy-carbamate (A.1.22), metyltetraprole (A.1.25), (*Z,*2*E*)-5-[1-(2,4-dichlorophenyl)pyrazol-3-yl]-oxy-2-methoxyimino-*N*,3-dimethyl-pent-3-enamide (A.1.34), (*Z,*2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methyl ester (A. 1.38);
   - inhibitors of complex III at Qᵢ site: cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5), [(1*S*,2*S*)-2-(4-fluoro-2-methyl-phenyl)-1,3-dimethyl-butyl] (2*S*)-2-[(3-hydroxy-4-methoxy-pyridine-2-carbonyl)amino]propanoate, [(1*S*,2*S*)-2-(2,4-dimethylphenyl)-1,3-dimethyl-butyl] (2*S*)-2-[(3-hydroxy-4-methoxy-pyridine-2-carbonyl)amino]propanoate,
      [(1*S*,2*S*)-2-(2,4-difluorophenyl)-1,3-dimethyl-butyl] (2*S*)-2-[(3-hydroxy-4-methoxy-pyridine-2-carbonyl)amino]propanoate, [(1*S*,2*S*)-2-(2-fluoro-4-methyl-phenyl)-1,3-dimethyl-butyl] (2*S*)-2-[(3-hydroxy-4-methoxy-pyridine-2-carbonyl)amino]propanoate, [(1*S*,2*S*)-2-(4-fluoro-2-methyl-phenyl)-1,3-dimethyl-butyl] (2*S*)-2-[(3-acetoxy-4-methoxy-pyridine-2-carbonyl)amino]propanoate, [(1*S*,2*S*)-2-(2,4-dimethylphenyl)-1,3-dimethyl-butyl] (2*S*)-2-[(3-acetoxy-4-methoxy-pyridine-2-carbonyl)amino]propanoate, [(1*S*,2*S*)-2-(2,4-difluorophenyl)-1,3-dimethyl-butyl] (2*S*)-2-[(3-acetoxy-4-methoxy-pyridine-2-carbonyl)amino]propanoate, [(1*S*,2*S*)-2-(2-fluoro-4-methylphenyl)-1,3-dimethyl-butyl] (2*S*)-2-[(3-acetoxy-4-methoxy-pyridine-2-carbonyl)amino]propanoate, [2-[[(1*S*)-2-[(1*S*,2*S*)-2-(4-fluoro-2-methyl-phenyl)-1,3-dimethyl-butoxy]-1-methyl-2-oxoethyl]carbamoyl]-4-methoxy-3-pyridyl]oxymethyl 2-methylpropanoate, [2-[[(1*S*)-2-[(1*S*,2*S*)-2-(2,4-dimethylphenyl)-1,3-dimethyl-butoxy]-1-methyl-2-oxo-ethyl]carbamoyl]-4-methoxy-3-pyridyl]oxymethyl 2-methylpropanoate,
      [2-[[(1*S*)-2-[(1*S*,2*S*)-2-(2,4-difluorophenyl)-1,3-dimethyl-butoxy]-1-methyl-2-oxo-ethyl]carbamoyl]-4-methoxy-3-pyridyl]oxymethyl 2-methylpropanoate, [2-[[(1*S*)-2-[(1*S*,2*S*)-2-(2-fluoro-4-methyl-phenyl)-1,3-dimethyl-butoxy]-1-methyl-2-oxo-ethyl]carbamoyl]-4-methoxy-3-pyridyl]oxymethyl 2-methylpropanoate, [(1*S*,2*S*)-1-methyl-2-(o-tolyl)propyl] (2*S*)-2-[(3-acetoxy-4-methoxy-pyridine-2-carbonyl)amino]propanoate, [(1*S*,2*S*)-1-methyl-2-(*o*-tolyl)propyl] (2*S*)-2-[(4-methoxy-3-propanoyloxy-pyridine-2-carbonyl)amino]propanoate, [(1*S*,2*S*)-1-methyl-2-(*o*-tolyl)propyl] (2*S*)-2-[(3-hydroxy-4-methoxy-pyridine-2-carbonyl)amino]propanoate, [4-methoxy-2-[[(1*S*)-1-methyl-2-[(1*S*,2*S*)-1-methyl-2-(*o*-tolyl)propoxy]-2-oxo-ethyl]carbamoyl]-3-pyridyl] 2-methylpropanoate, [(1*S*,2*S*)-2-(2,4-dimethylphenyl)-1-methyl-propyl] (2*S*)-2-[(3-acetoxy-4-methoxy-pyridine-2-carbonyl)amino]propanoate,
      [2-[[(1*S*)-2-[(1*S*,2*S*)-2-(2,4-dimethylphenyl)-1-methyl-propoxy]-1-methyl-2-oxo-ethyl]carbamoyl]-4-methoxy-3-pyridyl] 2-methylpropanoate, [(1*S*,2*S*)-2-(2,4-dimethylphenyl)-1-methyl-propyl]
      (2*S*)-2-[(3-hydroxy-4-methoxy-pyridine-2-carbonyl)amino]propanoate,
      [(1*S*,2*S*)-2-(2,6-dimethylphenyl)-1-methyl-propyl] (2*S*)-2-[(3-acetoxy-4-methoxy-pyridine-2-carbonyl)amino]propanoate, [2-[[(1*S*)-2-[(1*S*,2*S*)-2-(2,6-dimethylphenyl)-1-methyl-propoxy]-1-methyl-2-oxo-ethyl]carbamoyl]-4-methoxy-3-pyridyl] 2-methylpropanoate, [(1*S*,2*S*)-2-(2,6-dimethylphenyl)-1-methyl-propyl] (2*S*)-2-[(3-hydroxy-4-methoxy-pyridine-2-carbonyl)amino]propanoate, [(1*S*,2*S*)-2-[4-fluoro-2-(trifluoromethyl)phenyl]-1-methyl-propyl]
      (2*S*)-2-[(3-acetoxy-4-methoxy-pyridine-2-carbonyl)amino]propanoate,
      [2-[[(1*S*)-2-[(1*S*,2*S*)-2-[4-fluoro-2-(trifluoromethyl)phenyl]-1-methyl-propoxy]-1-methyl-2-oxoethyl]carbamoyl]-4-methoxy-3-pyridyl] 2-methylpropanoate, [(1*S*,2*S*)-2-[4-fluoro-2-(trifluoromethyl)phenyl]-1-methyl-propyl] (2*S*)-2-[(3-hydroxy-4-methoxy-pyridine-2-carbonyl)amino]propanoate, [(1*S*,2*S*)-2-(4-fluoro-2-methyl-phenyl)-1-methyl-propyl] (2*S*)-2-[(3-acetoxy-4-methoxy-pyridine-2-carbonyl)amino]propanoate, [2-[[(1*S*)-2-[(1*S*,2*S*)-2-(4-fluoro-2-methyl-phenyl)-1-methylpropoxy]-1-methyl-2-oxo-ethyl]carbamoyl]-4-methoxy-3-pyridyl] 2-methylpropanoate, [(1*S*,2*S*)-2-(4-fluoro-2-methyl-phenyl)-1-methyl-propyl] (2*S*)-2-[(3-hydroxy-4-methoxy-pyridine-2-carbonyl)amino]propanoate, [(1*S*,2*S*)-1-methyl-2-[2-(trifluoromethyl)phenyl]propyl] (2*S*)-2-[(3-acetoxy-4-methoxy-pyridine-2-carbonyl)amino]propanoate, [4-methoxy-2-[[(1*S*)-1-methyl-2-[(1*S*,2*S*)-1-methyl-2-[2-(trifluoromethyl)phenyl]propoxy]-2-oxo-ethyl]carbamoyl]-3-pyridyl] 2-methylpropanoate, [(1*S*,2*S*)-1-methyl-2-[2-(trifluoromethyl)phenyl]propyl] (2*S*)-2-[(3-hydroxy-4-methoxy-pyridine-2-carbonyl)amino]propanoate, [(1*S*,2*S*)-2-(4-fluoro-2,6-dimethyl-phenyl)-1-methyl-propyl] (2*S*)-2-[(3-acetoxy-4-methoxy-pyridine-2-carbonyl)amino]propanoate, [2-[[(1*S*)-2-[(1*S*,2*S*)-2-(4-fluoro-2,6-dimethyl-phenyl)-1-methyl-propoxy]-1-methyl-2-oxo-ethyl]carbamoyl]-4-methoxy-3-pyridyl] 2-methylpropanoate, [(1*S*,2*S*)-2-(4-fluoro-2,6-dimethyl-phenyl)-1-methyl-propyl] (2*S*)-2-[(3-hydroxy-4-methoxy-pyridine-2-carbonyl)amino]propanoate;
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*)-3-ethyl-1,1-dimethyl-indan-4-yl]pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]pyridine-3-carboxamide (A.3.37), 2-(difluoromethyl)-*N-*(3-isobutyl-1,1-dimethyl-indan-4-yl)pyridine-3-carboxamide (A.3.38), 2-(difluoromethyl)-*N*-[(3*R*)-3-isobutyl-1,1-dimethyl-indan-4-yl]pyridine-3-carboxamide (A.3.39);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), 4-[[6-[2-(2,4-difluorophenyl)-1,1-difluoro-2-hydroxy-3-(5-sulfanyl-1,2,4-triazol-1-yl)propyl]-3-pyridyl]oxy]benzonitrile (B.1.33), ipfentrifluconazole (B.1.37), mefentrifluconazole (B.1.38), (2*R*)-2-[4-(4-chlorophenoxy)-2-(trifluoromethyl)phenyl]-1-(1,2,4-triazol-1-yl)propan-2-ol, (2*S*)-2-[4-(4-chlorophenoxy)-2-(trifluoromethyl)phenyl]-1-(1,2,4-triazol-1-yl)propan-2-ol, 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluoro-phenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (1.2.2), carpropamid (1.2.3), dicyclomet (1.2.4), fenoxanil (1.2.5);
J) Plant defense inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxin-copper (K.1.22), proquinazid (K.1.23), tebufloquin (K.1.24), tecloftalam (K.1.25), triazoxide (K.1.26), *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N*-ethyl-*N*-methyl formamidine (K.1.28), *N*'-[4-[[3-[(4-chlorophenyl)methyl]-1,2,4-thiadiazol-5-yl]oxy]-2,5-dimethylphenyl]-*N*-ethyl-*N*-methyl-formamidine (K.1.29), *N*'-(5-bromo-6-indan-2-yloxy-2-methyl-3-pyridyl)-*N*-ethyl-*N*-methyl-formamidine (K.1.30), *N*'-[5-bromo-6-[1-(3,5-difluorophenyl)ethoxy]-2-methyl-3-pyridyl]-*N*-ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), *N*'-[5-bromo-2-methyl-6-(1-phenylethoxy)-3-pyridyl]-*N*-ethyl-*N*-methyl-formamidine (K.1.33), *N*'-(2-methyl-5-trifluoromethyl-4-(3-trimethylsilanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (*Z*)-3-amino-2-cyano-3-phenyl-prop-2-enoate (K.1.40), picarbutrazox (K.1.41), pentyl *N*-[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]-quinazoline (K.1.51), dichlobentiazox (K.1.52), *N*'-(2,5-dimethyl-4-phenoxy-phenyl)-*N*-ethyl-*N-*methyl-formamidine (K.1.53), aminopyrifen (K.1.54), fluopimomide (K.1.55), *N*'-[5-bromo-2-methyl-6-(1-methyl-2-propoxy-ethoxy)-3-pyridyl]-N-ethyl-N-methyl-formamidine (K.1.56), N'-[4-(4,5-dichlorothiazol-2-yl)oxy-2,5-dimethyl-phenyl]-N-ethyl-N-methyl-formamidine (K.1.57), N-(2-fluorophenyl)-4-[5-(trifluoromethyl)-1,2,4-oxadiazol-3-yl]benzamide (K.1.58), N-methyl-4-[5-(trifluoromethyl)-1,2,4-oxadiazol-3-yl]benzenecarbothioamide (K.1.59);
L) Biopesticides
   L4) Biochemical pesticides with insecticidal, acaricidal, molluscidal, pheromone and/or nematicidal activity: L-carvone, citral, (*E,Z*)-7,9-dodecadien-1-yl acetate, ethyl formate, (*E,Z*)-2,4-ethyl decadienoate (pear ester), (*Z,Z,E*)-7,11,13-hexadecathena!, heptyl butyrate, isopropyl myristate, lavanulyl senecioate, cis-jasmone, 2-methyl 1-butanol, methyl eugenol, methyl jasmonate, (*E,Z*)-2,13-octadecadien-1-ol, (*E,Z*)-2,13-octadecadien-1-ol acetate, (*E,Z*)-3,13-octadecadien-1-ol, (*R*)-1-octen-3-ol, pentatermanone, (*E,Z,Z*)-3,8,11-tetradecathenyl acetate, (*Z,E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract;

Preferred fungicides are the following: (3-ethoxypropyl)mercury bromide, 2-methoxyethyl¬mercury chloride, 2-phenylphenol, 8-hydroxyquinoline sulfate, 8-phenylmercurioxy¬quinoline, acibenzolar, acylamino acid fungicides, acypetacs, aldimorph, aliphatic nitrogen fungicides, allyl alcohol, amide fungicides, ampropylfos, anilazine, anilide fungicides, antibiotic fungicides, aromatic fungicides, aureofungin, azaconazole, azithiram, azoxystrobin, barium polysulfide, benalaxyl benalaxyl-M, benodanil, benomyl, benquinox, bentaluron, benthiavalicarb, benzalkonium chloride, benzamacril, benzamide fungicides, benzamorf, benzanilide fungicides, benzimidazole fungicides, benzimidazole precursor fungicides, benzimidazolylcarbamate fungicides, benzohydroxamic acid, benzothiazole fungicides, bethoxazin, binapacryl, biphenyl, bitertanol, bithionol, blasticidin-S, Bordeaux mixture, boscalid, bridged diphenyl fungicides, bromuconazole, bupirimate, Burgundy mixture, buthiobate, butylamine, calcium polysulfide, captafol, captan, carbamate fungicides, carbamorph, carbanilate fungicides, carbendazim, carboxin, carpropamid, carvone, Cheshunt mixture, chinomethionat, chlobenthiazone, chloraniformethan, chloranil, chlorfenazole, chlorodinitronaphthalene, chloroneb, chloropicrin, chlorothalonil, chlorquinox, chlozolinate, ciclopirox, climbazole, clotrimazole, conazole fungicides, conazole fungicides (imidazoles), conazole fungicides (triazoles), copper(II) acetate, copper(II) carbonate, basic, copper fungicides, copper hydroxide, copper naphthenate, copper oleate, copper oxychloride, copper(II) sulfate, copper sulfate, basic, copper zinc chromate, cresol, cufraneb, cuprobam, cuprous oxide, cyazofamid, cyclafuramid, cyclic dithiocarbamate fungicides, cycloheximide, cyflufenamid, cymoxanil, cypendazole, cyproconazole, cyprodinil, dazomet, DBCP, debacarb, decafentin, dehydroacetic acid, dicarboximide fungicides, dichlofluanid, dichlone, dichlorophen, dichlorophenyl, dicarboximide fungicides, dichlozoline, diclobutrazol, diclocymet, diclomezine, dicloran, diethofencarb, diethyl pyro carbonate, difenoconazole, diflumetorim, dimethirimol, dimethomorph, dimoxystrobin, diniconazole, dinitrophenol fungicides, dinobuton, dinocap, dinocton, dinopenton, dinosulfon, dinoterbon, diphenylamine, dipyrithione, disulfiram, ditalimfos, dithianon, dithiocarbamate fungicides, DNOC, dodemorph, dodicin, dodine, DONATODINE, drazoxolon, edifenphos, epoxiconazole, etaconazole, etem, ethaboxam, ethirimol, ethoxyquin, ethylmercury 2,3-dihydroxypropyl mercaptide, ethylmercury acetate, ethylmercury bromide, ethylmercury chloride, ethylmercury phosphate, etridiazole, famoxadone, fenamidone, fenaminosulf, fenapanil, fenarimol, fenbuconazole, fenfuram, fenhexamid, fenitropan, fenoxanil, fenpiclonil, fenpropidin, fenpropimorph, fentin, ferbam, ferimzone, fluazinam, fludioxonil, flumetover, flumorph, fluopicolide, fluoroimide, fluotrimazole, fluoxastrobin, fluquinconazole, flusilazole, flusulfamide, flutolanil, flutriafol, folpet, formaldehyde, fosetyl, fuberidazole, furalaxyl, furametpyr, furamide fungicides, furanilide fungicides, furcarbanil, furconazole, furconazole-cis, furfural, furmecyclox, furophanate, glyodin, griseofulvin, guazatine, halacrinate, hexachlorobenzene, hexachlorobutadiene, hexachlorophene, hexaconazole, hexylthiofos, hydrargaphen, hymexazol, imazalil, imibenconazole, imidazole fungicides, iminoctadine, inorganic fungicides, inorganic mercury fungicides, iodomethane, ipconazole, iprobenfos, iprodione, iprovalicarb, isoprothiolane, isovaledione, kasugamycin, kresoxim-methyl, lime sulphur, mancopper, mancozeb, maneb, mebenil, mecarbinzid, mepanipyrim, mepronil, mercuric chloride, mercuric oxide, mercurous chloride, mercury fungicides, metalaxyl, metalaxyl-M, metam, metazoxolon, metconazole, methasulfocarb, methfuroxam, methyl bromide, methyl isothiocyanate, methylmercury benzoate, methylmercury dicyandiamide, methylmercury pentachlorophenoxide, metiram, metominostrobin, metrafenone, metsulfovax, milneb, morpholine fungicides, myclobutanil, myclozolin, N-(ethylmercury)-p-toluenesulphonanilide, nabam, natamycin, nitrostyrene, nitrothal-isopropyl, nuarimol, OCH, octhilinone, ofurace, organomercury fungicides, organophosphorus fungicides, organotin fungicides, orysastrobin, oxadixyl, oxathiin fungicides, oxazole fungicides, oxine copper, oxpoconazole, oxycarboxin, pefurazoate, penconazole, pencycuron, pentachlorophenol, penthiopyrad, phenylmercuriurea, phenylmercury acetate, phenylmercury chloride, phenylmercury derivative of pyrocatechol, phenylmercury nitrate, phenylmercury salicylate, phenylsulfamide fungicides, phosdiphen, phthalide, phthalimide fungicides, picoxystrobin, piperalin, polycarbamate, polymeric dithiocarbamate fungicides, polyoxins, polyoxorim, polysulfide fungicides, potassium azide, potassium polysulfide, potassium thiocyanate, probenazole, prochloraz, procymidone, propamocarb, propiconazole, propineb, proquinazid, prothiocarb, prothioconazole, pyracarbolid, pyraclostrobin, pyrazole fungicides, pyrazophos, pyridine fungicides, pyridinitril, pyrifenox, pyrimethanil, pyrimidine fungicides, pyroquilon, pyroxychlor, pyroxyfur, pyrrole fungicides, quinacetol, quinazamid, quinconazole, quinoline fungicides, quinone fungicides, quinoxaline fungicides, quinoxyfen, quintozene, rabenzazole, salicylanilide, silthiofam, simeconazole, sodium azide, sodium orthophenylphenoxide, sodium pentachlorophenoxide, sodium polysulfide, spiroxamine, streptomycin, strobilurin fungicides, sulfonanilide fungicides, sulfur, sultropen, TCMTB, tebuconazole, tecloftalam, tecnazene, tecoram, tetraconazole, thiabendazole, thiadifluor, thiazole fungicides, thicyofen, thifluzamide, thiocarbamate fungicides, thiochlorfenphim, thiomersal, thiophanate, thiophanate-methyl, thiophene fungicides, thioquinox, thiram, tiadinil, tioxymid, tivedo, tolclofos-methyl, tolnaftate, tolylfluanid, tolylmercury acetate, triadimefon, triadimenol, triamiphos, triarimol, triazbutil, triazine fungicides, triazole fungicides, triazoxide, tributyltin oxide, trichlamide, tricyclazole, tridemorph, trifloxystrobin, triflumizole, triforine, mefentrifluconazole, metyltetraprole, triticonazole, unclassified fungicides, undecylenic acid, uniconazole, urea fungicides, validamycin, valinamide fungicides, vinclozolin, zarilamid, zinc naphthenate, zineb, ziram, zoxamide and their mixtures.
Herbicides: An herbicide is a pesticide used to kill unwanted plants. Selective herbicides kill specific targets while leaving the desired crop relatively unharmed. Some of these act by interfering with the growth of the weed and are often based on plant hormones. Herbicides used to clear waste ground are nonselective and kill all plant material with which they come into contact. Herbicides are widely used in agriculture and in landscape turf management. They are applied in total vegetation control (TVC) programs for maintenance of highways and railroads. Smaller quantities are used in forestry, pasture systems, and management of areas set aside as wildlife habitat. In the following, a number of suitable herbicides are compiled:

In one embodiment, the microparticles or compositions of the invention can contain a large number of representatives of other groups of herbicidal or growth-regulatory active substances or their mixtures as the active substance, below referred to as herbicidal or growth-regulatory active substances B) and C), or applied together with these. It is also possible that the microparticles may comprise one or more active substances B) or C) encapsulated in the microparticle.
B) herbicides of class b1) to b15):
   b1) lipid biosynthesis inhibitors;
   b2) acetolactate synthase inhibitors (ALS inhibitors);
   b3) photosynthesis inhibitors;
   b4) protoporphyrinogen-IX oxidase inhibitors,
   b5) bleacher herbicides;
   b6) enolpyruvyl shikimate 3-phosphate synthase inhibitors (EPSP inhibitors);
   b7) glutamine synthetase inhibitors;
   b8) 7,8-dihydropteroate synthase inhibitors (DHP inhibitors);
   b9) mitosis inhibitors;
   b10) inhibitors of the synthesis of very long chain fatty acids (VLCFA inhibitors);
   b11) cellulose biosynthesis inhibitors;
   b12) decoupler herbicides;
   b13) auxinic herbicides;
   b14) auxin transport inhibitors; and
   b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin, methyl azide, methyl bromide, methyl-dymron, methyl iodide, MSMA, oleic acid, oxaziclomefone, pelargonic acid, pyributicarb, quinoclamine, tetflupyrolimet, triaziflam, tridiphane and 6-chloro-3-(2-cyclopropyl-6-methylphenoxy)-4-pyridazinol (CAS 499223-49-3) and its salts and esters;
   including their agriculturally acceptable salts or derivatives;
   and
C) safeners.

If the herbicidal compounds B and/or the safeners C as described herein are capable of forming geometrical isomers, for example E/Z isomers, it is possible to use both, the pure isomers and mixtures thereof, in the compositions according to the invention.

If the herbicidal compounds B and/or the safeners C as described herein have one or more centers of chirality and, as a consequence, are present as enantiomers or diastereomers, it is possible to use both, the pure enantiomers and diastereomers and their mixtures, in the compositions according to the invention.

If the herbicidal compounds B and/or the safeners C as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropyl ammonium, dimethylammonium, diethylammonium, diisopropylammonium, trimethylammonium, triethylammonium, tris(isopropyl)ammonium, heptylammonium, dodecyl ammonium, tetradecyl ammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyl trimethylammonium, benzyl triethylammonium, N,N,N-trimethylethanolammonium (choline salt), furthermore phosphonium ions, sulfonium ions, preferably tri(C₁-C₄-alkyl)sulfonium, such as trimethyl sulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogen sulfate, methyl sulfate, sulfate, dihydrogen phosphate, hydrogen phosphate, nitrate, bicarbonate, carbonate, hexafluoro silicate, hexafluorophosphate, benzoate and also the anions of C₁-C₄-alkanoic acids, preferably formate, acetate, propionate and butyrate.

Herbicidal compounds B and/or safeners C as described herein having a carboxyl group can be employed in the form of the acid, in the form of an agriculturally suitable salt as mentioned above or else in the form of an agriculturally acceptable derivative, for example as amides, such as mono- and di-C₁-C₆-alkylamides or arylamides, as esters, for example as allyl esters, propargyl esters, C₁-C₁₀-alkyl esters, alkoxyalkyl esters, tefuryl ((tetrahydrofuran-2-yl)methyl) esters and also as thioesters, for example as C₁-C₁₀-alkylthio esters. Preferred mono- and di-C₁-C₆-alkylamides are the methyl and the dimethylamides. Preferred arylamides are, for example, the anilides and the 2-chloroanilides. Preferred alkyl esters are, for example, the methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄-alkoxy-C₁-C₄-alkyl esters are the straight-chain or branched C₁-C₄-alkoxy ethyl esters, for example the 2-methoxyethyl, 2-ethoxyethyl, 2-butoxyethyl (butotyl), 2-butoxypropyl or 3-butoxypropyl ester. An example of a straight-chain or branched C₁-C₁₀-alkylthio ester is the ethylthio ester.

According to a first embodiment of the invention the compositions contain at least one inhibitor of the lipid biosynthesis (herbicide b1). These are compounds that inhibit lipid biosynthesis. Inhibition of the lipid biosynthesis can be affected either through inhibition of acetylCoA carboxylase (hereinafter termed ACC herbicides) or through a different mode of action (hereinafter termed non-ACC herbicides). The ACC herbicides belong to the group A of the HRAC classification system whereas the non-ACC herbicides belong to the group N of the HRAC classification.

According to a second embodiment of the invention the compositions contain at least one ALS inhibitor (herbicide b2). The herbicidal activity of these compounds is based on the inhibition of acetolactate synthase and thus on the inhibition of the branched chain amino acid biosynthesis. These inhibitors belong to the group B of the HRAC classification system.

According to a third embodiment of the invention the compositions contain at least one inhibitor of photosynthesis (herbicide b3). The herbicidal activity of these compounds is based either on the inhibition of the photosystem II in plants (so-called PSII inhibitors, groups C1, C2 and C3 of HRAC classification) or on diverting the electron transfer in photosystem I in plants (so-called PSI inhibitors, group D of HRAC classification) and thus on an inhibition of photosynthesis. Amongst these, PSII inhibitors are preferred.

According to a fourth embodiment of the invention the compositions contain at least one inhibitor of protoporphyrinogen-IX-oxidase (herbicide b4). The herbicidal activity of these compounds is based on the inhibition of the protoporphyrinogen-IX-oxidase. These inhibitors belong to the group E of the HRAC classification system.

According to a fifth embodiment of the invention the compositions contain at least one bleacher-herbicide (herbicide b5). The herbicidal activity of these compounds is based on the inhibition of the carotenoid biosynthesis. These include compounds which inhibit carotenoid biosynthesis by inhibition of phytoene desaturase (so-called PDS inhibitors, group F1 of HRAC classification), compounds that inhibit the 4-hydroxyphenylpyruvate-dioxygenase (HPPD inhibitors, group F2 of HRAC classification), compounds that inhibit DOXsynthase (group F4 of HRAC class) and compounds which inhibit carotenoid biosynthesis by an unknown mode of action (bleacher - unknown target, group F3 of HRAC classification).

According to a sixth embodiment of the invention the compositions contain at least one EPSP synthase inhibitor (herbicide b6). The herbicidal activity of these compounds is based on the inhibition of enolpyruvyl shikimate 3-phosphate synthase, and thus on the inhibition of the amino acid biosynthesis in plants. These inhibitors belong to the group G of the HRAC classification system.

According to a seventh embodiment of the invention the compositions contain at least one glutamine synthetase inhibitor (herbicide b7). The herbicidal activity of these compounds is based on the inhibition of glutamine synthetase, and thus on the inhibition of the amino acid biosynthesis in plants. These inhibitors belong to the group H of the HRAC classification system.

According to an eighth embodiment of the invention the compositions contain at least one DHP synthase inhibitor (herbicide b8). The herbicidal activity of these compounds is based on the inhibition of 7,8-dihydropteroate synthase. These inhibitors belong to the group I of the HRAC classification system.

According to a ninth embodiment of the invention the compositions contain at least one mitosis inhibitor (herbicide b9). The herbicidal activity of these compounds is based on the disturbance or inhibition of microtubule formation or organization, and thus on the inhibition of mitosis. These inhibitors belong to the groups K1 and K2 of the HRAC classification system. Among these, compounds of the group K1, in particular dinitroanilines, are preferred.

According to a tenth embodiment of the invention the compositions contain at least one VLCFA inhibitor (herbicide b10). The herbicidal activity of these compounds is based on the inhibition of the synthesis of very long chain fatty acids and thus on the disturbance or inhibition of cell division in plants. These inhibitors belong to the group K3 of the HRAC classification system.

According to an eleventh embodiment of the invention the compositions contain at least one cellulose biosynthesis inhibitor (herbicide b11). The herbicidal activity of these compounds is based on the inhibition of the biosynthesis of cellulose and thus on the inhibition of the synthesis of cell walls in plants. These inhibitors belong to the group L of the HRAC classification system.

According to a twelfth embodiment of the invention the compositions contain at least one decoupler herbicide (herbicide b12). The herbicidal activity of these compounds is based on the disruption of the cell membrane. These inhibitors belong to the group M of the HRAC classification system.

According to a thirteenth embodiment of the invention the compositions contain at least one auxinic herbicide (herbicide b13). These include compounds that mimic auxins, i.e. plant hormones, and affect the growth of the plants. These compounds belong to the group O of the HRAC classification system.

According to a fourteenth embodiment of the invention the compositions contain at least one auxin transport inhibitor (herbicide b14). The herbicidal activity of these compounds is based on the inhibition of the auxin transport in plants. These compounds belong to the group P of the HRAC classification system.

As to the given mechanisms of action and classification of the active substances, see e.g. "HRAC, Classification of Herbicides According to Mode of Action", http://www.plantprotection.org/hrac/MOA.html).

Preference is given to those compositions according to the present invention comprising at least one herbicide B selected from herbicides of class b2, b3, b4, b5, b6, b9 and b10.

Specific preference is given to those compositions according to the present invention which comprise at least one herbicide B selected from the herbicides of class b4, b6 b9 and b10.

Particular preference is given to those compositions according to the present invention which comprise at least one herbicide B selected from the herbicides of class b4, b6 and b10.

Examples of herbicides B which can be used in combination with the compositions according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim,
   4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
   imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam,
   pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
   sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
   among these, a preferred embodiment of the invention relates to those compositions comprising at least one imidazolinone herbicide;
b3) from the group of the photosynthesis inhibitors:
   amicarbazone, inhibitors of the photosystem II, e.g. 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-pyrazol-3-yl)-3-chloro-2-hydroxy-4-methyl-2H-pyrrol-5-one (CAS 1654747-80-44-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8), 1-(5-tert-butylisoxazol-3-yl)-4-ethoxy-5-hydroxy-3-methyl-imidazolidin-2-one (CAS 1844836-64-1), triazine herbicides, including of chlorotriazine, triazinones, triazindiones, methylthiotriazines and pyridazinones such as ametryn, atrazine, chloridazone, cyanazine, desmetryn, dimethametryn, hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, chlorphthalim, cinidon-ethyl, cyclopyranil, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), methyl (*E*)-4-[2-chloro-5-[4-chloro-5-(difluoromethoxy)-1*H*-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4),
   2-[2-chloro-5-[3-chloro-5-(trifluoromethyl)-2-pyridinyl]-4-fluorophenoxy]-2-methoxy-acetic acid methyl ester (CAS 1970221-16-9), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-acetic acid methyl ester (CAS 2158274-96-3), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy] acetic acid ethyl ester (CAS 158274-50-9), methyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2271389-22-9), ethyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2230679-62-4), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-acetic acid methyl ester (CAS 2158275-73-9), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy] acetic acid ethyl ester (CAS 2158274-56-5), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-N-(methylsulfonyl)-acetamide (CAS 2158274-53-2), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-N-(methylsulfonyl)-acetamide (CAS 2158276-22-1);
b5) from the group of the bleacher herbicides:
   PDS inhibitors: beflubutamid, diflufenican, fluridone, flurochloridone, flurtamone, norflurazon, picolinafen, 4-(3-trifluoromethylphenoxy)-2-(4-trifluoromethylphenyl)pyrimidine (CAS 180608-33-7) and 3-chloro-2-[-3-(difluoromethyl)isoxazol-5-yl]phenyl-5-chloropyrimidin-2-yl-ether, HPPD inhibitors: benzobicyclon, benzofenap, bicyclopyrone, clomazone, fenquinotrione, isoxaflutole, mesotrione, oxotrione (CAS 1486617-21-3), pyrasulfotole, pyrazolynate, pyrazoxyfen, sulcotrione, tefuryltrione, tembotrione, tolpyralate, topramezone, bipyrazone, fenpyrazone, cypyrafluone, tripyrasulfone, benquinotrione, 2-(3,4-dimethoxyphenyl)-4-[2-hydroxy-6-oxocyclohex-1-en-1yl)carbonyl]-6-methylpyridazin-3(2*H*)-one; bleacher, unknown target: aclonifen, amitrole flumeturon,2-chloro-3-methylsulfanyl-N-(1-methyltetrazol-5-yl)-4-(trifluoromethyl)benzamide (CAS 1361139-71-0), bixlozone and 2-(2,5-dichlorophenyl)methyl-4,4-dimethyl-3-isoxazolidinone (CAS 81778-66-7);
b8) from the group of the DHP synthase inhibitors:
   asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;
b10) from the group of the VLCFA inhibitors:
   chloroacetamides such as acetochlor, alachlor, amidochlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide, napropamide and napropamide-M, tetrazolinones such fentrazamide, and other herbicides such as anilofos, cafenstrole, fenoxasulfone, ipfencarbazone, piperophos, pyroxasulfone, dimesulfazet and isoxazoline compounds of the formulae II.1, II.2, II.3, II.4, II.5, II.6, II.7, II.8 and II.9 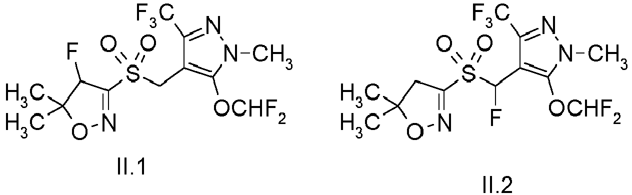 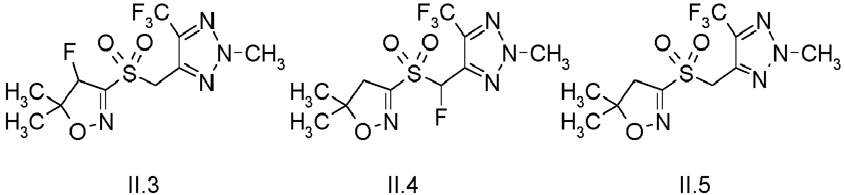 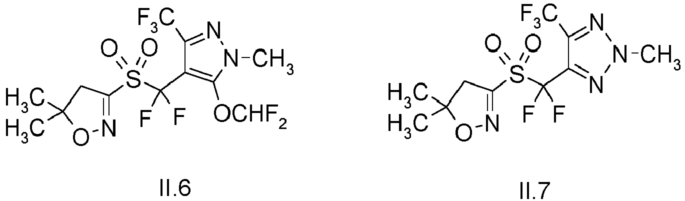 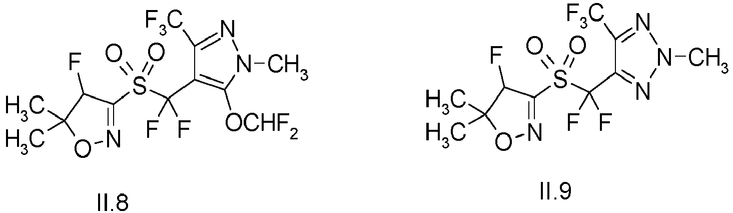 the isoxazoline compounds of the formula (II) are known in the art, e.g. from WO 2006/024820, WO 2006/037945, WO 2007/071900 and WO 2007/096576;
   among the VLCFA inhibitors, preference is given to chloroacetamides and oxyacetamides;
b11) from the group of the cellulose biosynthesis inhibitors:
   chlorthiamid, dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters such as clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dichlorprop and its salts and esters, dichlorprop-P and its salts and esters, flopyrauxifen, fluroxypyr, fluroxypyr-butometyl, fluroxypyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin, methyl azide, methyl bromide, methyl-dymron, methyl iodide, MSMA, oleic acid, oxaziclomefone, pelargonic acid, pyributicarb, quinoclamine, tetflupyrolimet and tridiphane.

Preferred herbicides B that can be used in combination with the compositions according to the present invention are:
b1) from the group of the lipid biosynthesis inhibitors:
   clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, dimepiperate, EPTC, esprocarb, ethofumesate, molinate, orbencarb, prosulfocarb, thiobencarb and triallate;
b2) from the group of the ALS inhibitors:
   amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisulfuron, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl, tritosulfuron and triafamone;
b3) from the group of the photosynthesis inhibitors:
   ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine, thidiazuron, 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-pyrazol-3-yl)-3-chloro-2-hydroxy-4-methyl-2H-pyrrol-5-one (CAS 1654747-80-4), 4-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8) and 1-(5-tert-butylisoxazol-3-yl)-4-ethoxy-5-hydroxy-3-methyl-imidazolidin-2-one (CAS 1844836-64-1);
b4) from the group of the protoporphyrinogen-IX oxidase inhibitors:
   acifluorfen-sodium, bencarbazone, benzfendizone, butafenacil, carfentrazone-ethyl, cinidon-ethyl, cyclopyranil, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen, pyraflufen-ethyl, saflufenacil, sulfentrazone, tiafenacil, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0) ;1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4),
   2-[2-chloro-5-[3-chloro-5-(trifluoromethyl)-2-pyridinyl]-4-fluorophenoxy]-2-methoxy-acetic acid methyl ester (CAS 1970221-16-9), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-acetic acid methyl ester (CAS 2158274-96-3), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy] acetic acid ethyl ester (CAS 158274-50-9), methyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2271389-22-9), ethyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2230679-62-4), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-acetic acid methyl ester (CAS 2158275-73-9), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy] acetic acid ethyl ester (CAS 2158274-56-5), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-N-(methylsulfonyl)-acetamide (CAS 2158274-53-2), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-N-(methylsulfonyl)-acetamide(CAS 2158276-22-1);
b5) from the group of the bleacher herbicides:
   aclonifen, amitrole, beflubutamid, benzobicyclon, bicyclopyrone, clomazone, cypyrafluone, diflufenican, fenquinotrione, flumeturon, flurochloridone, flurtamone, isoxaflutole, mesotrione, oxotrione (CAS 1486617-21-3), norflurazon, picolinafen, pyrasulfotole, pyrazolynate, sulcotrione, tefuryltrione, tembotrione, tolpyralate, topramezone, 4-(3-trifluoromethylphenoxy)-2-(4-trifluoromethylphenyl)pyrimidine (CAS 180608-33-7), 2-chloro-3-methylsulfanyl-N-(1-methyltetrazol-5-yl)-4-(trifluoromethyl)benzamide (CAS 1361139-71-0), bixlozone, 2-(2,5-dichlorophenyl)methyl-4,4-dimethyl-3-isoxazolidinone (CAS 81778-66-7), chloro-2-[-3-(difluoromethyl)isoxazol-5-yl]phenyl-5-chloropyrimidin-2-yl-ether and 2-(3,4-dimethoxyphenyl)-4-[2-hydroxy-6-oxocyclohex-1-en-1yl)carbonyl]-6-methylpyridazin-3(*2H*)-one;
b8) from the group of the DHP synthase inhibitors: asulam;
b9) from the group of the mitosis inhibitors:
   benfluralin, dithiopyr, ethalfluralin, flamprop, flamprop-isopropyl, flamprop-methyl, flamprop-M-isopropyl, flamprop-M-methyl, oryzalin, pendimethalin, thiazopyr and trifluralin;
b10) from the group of the VLCFA inhibitors:
   acetochlor, alachlor, amidochlor, anilofos, butachlor, cafenstrole, dimethenamid, dimethenamid-P, dimesulfazet, fentrazamide, flufenacet, mefenacet, metazachlor, metolachlor, S-metolachlor, naproanilide, napropamide, napropamide-M, pretilachlor, fenoxasulfone, ipfencarbazone, pyroxasulfone, thenylchlor and isoxazoline-compounds of the formulae II.1, II.2, II.3, II.4, II.5, II.6, II.7, II.8 and II.9 as mentioned above;
b11) from the group of the cellulose biosynthesis inhibitors: dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b13) from the group of the auxinic herbicides:
   2,4-D and its salts and esters, aminocyclopyrachlor and its salts and esters, aminopyralid and its salts such as aminopyralid-dimethylammonium, aminopyralid-tris(2-hydroxypropyl)ammonium and its esters, clopyralid and its salts and esters, dichlorprop-P and its salts and esters, flopyrauxifen, fluroxypyr-meptyl, halauxifen and its salts and esters (CAS 943832-60-8, MCPA and its salts and esters, MCPB and its salts and esters, mecoprop-P and its salts and esters, picloram and its salts and esters, quinclorac, quinmerac, triclopyr and its salts and esters, florpyrauxifen , florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr and diflufenzopyr-sodium;
b15) from the group of the other herbicides: bromobutide, cinmethylin, cumyluron, cyclopyrimorate (CAS 499223-49-3) and its salts and esters, dalapon, difenzoquat, difenzoquat-metilsulfate, DSMA, dymron (= daimuron), indanofan, metam, methylbromide, MSMA, oxaziclomefone, pyributicarb, tetflupyrolimet and tridiphane.

Particularly preferred herbicides B that can be used in combination with the compositions according to the present invention are:
b1) from the group of the lipid biosynthesis inhibitors: clodinafop-propargyl, cycloxydim, cyhalofop-butyl, fenoxaprop-P-ethyl, pinoxaden, profoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); esprocarb, prosulfocarb, thiobencarb and triallate;
b2) from the group of the ALS inhibitors: bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, nicosulfuron, penoxsulam, propoxycarbazon-sodium, propyrisulfuron, pyrazosulfuron-ethyl, pyroxsulam, rimsulfuron, sulfosulfuron, thiencarbazon-methyl, tritosulfuron and triafamone;
b3) from the group of the photosynthesis inhibitors: ametryn, atrazine, diuron, fluometuron, hexazinone, isoproturon, linuron, metribuzin, paraquat, paraquat-dichloride, propanil, terbutryn, terbuthylazine, 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butylisoxazol-3-yl)-4-ethoxy-5-hydroxy-3-methyl-imidazolidin-2-one (CAS 1844836-64-1);
b4) from the group of the protoporphyrinogen-IX oxidase inhibitors: cyclopyranil, flumioxazin, oxyfluorfen, pyraflufen, pyraflufen-ethyl, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydroisoindole-1,3-dione (CAS 1300118-96-0), and 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0);
   2-[2-chloro-5-[3-chloro-5-(trifluoromethyl)-2-pyridinyl]-4-fluorophenoxy]-2-methoxy-acetic acid methyl ester (CAS 1970221-16-9), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-acetic acid methyl ester (CAS 2158274-96-3), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy] acetic acid ethyl ester (CAS 158274-50-9), methyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2271389-22-9), ethyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2230679-62-4), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-acetic acid methyl ester (CAS 2158275-73-9), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy] acetic acid ethyl ester (CAS 2158274-56-5), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-N-(methylsulfonyl)-acetamide (CAS 2158274-53-2), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-N-(methylsulfonyl)-acetamide (CAS 2158276-22-1);
b5) from the group of the bleacher herbicides: amitrole, bicyclopyrone, clomazone, diflufenican, fenquinotrione, flumeturon, flurochloridone, isoxaflutole, mesotrione, oxotrione (CAS 1486617-21-3), picolinafen, sulcotrione, tefuryltrione, tembotrione, tolpyralate, topramezone, 2-chloro-3-methylsulfanyl-N-(1-methyltetrazol-5-yl)-4-(trifluoromethyl)benzamide (CAS 1361139-71-0), bixlozone, 2-(2,5-dichlorophenyl)methyl-4,4-dimethyl-3-isoxazolidinone (CAS 81778-66-7), and chloro-2-[-3-(difluoromethyl)isoxazol-5-yl]phenyl-5-chloropyrimidin-2-yl-ether;
b9) from the group of the mitosis inhibitors: pendimethalin and trifluralin;
b10) from the group of the VLCFA inhibitors: acetochlor, cafenstrole, dimethenamid-P, fentrazamide, flufenacet, mefenacet, metazachlor, metolachlor, S-metolachlor, fenoxasulfone, ipfencarbazone and pyroxasulfone; likewise, preference is given to isoxazoline compounds of the formulae II.1, II.2, II.3, II.4, II.5, II.6, II.7, II.8 and II.9 as mentioned above;
b11) from the group of the cellulose biosynthesis inhibitors: indaziflam, isoxaben and triaziflam;
b13) from the group of the auxinic herbicides: 2,4-D and its salts and esters such as clacyfos, and aminocyclopyrachlor and its salts and esters, aminopyralid and its salts and its esters, clopyralid and its salts and esters, flopyrauxifen, fluroxypyr-meptyl, halauxifen, halauxifen-methyl, quinclorac, quinmerac, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr and diflufenzopyr-sodium,
b15) from the group of the other herbicides: cinmethylin, dymon (= daimuron), indanofan, oxaziclomefone and tetflupyrolimet.

In another embodiment of the present invention the compositions according to the present invention comprise at least one safener C.

Safeners are chemical compounds which prevent or reduce damage on useful plants without having a major impact on the herbicidal action of the herbicidal active components of the present compositions towards unwanted plants. They can be applied either before sowings (e.g. on seed treatments, shoots or seedlings) or in the pre-emergence application or post-emergence application of the useful plant. The safeners and the ***compositions*** of the invention and/or the herbicides B can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of preferred safeners C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4), metcamifen and BPCMS (CAS 54091-06-4).

Especially preferred safeners C are benoxacor, cloquintocet, cyprosulfamide, dichlormid, fenchlorazole, fenclorim, flurazole, fluxofenim, furilazole, isoxadifen, mefenpyr, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and metcamifen.

Particularly preferred safeners C are benoxacor, cloquintocet, cyprosulfamide, dichlormid, fenchlorazole, fenclorim, furilazole, isoxadifen, mefenpyr, naphthalic anhydride, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and metcamifen.

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

Active compounds B and C having a carboxyl group can be employed in the form of the acid, in the form of an agriculturally suitable salt as mentioned above or else in the form of an agriculturally acceptable derivative in the compositions according to the invention.

Suitable salts of 2,4-D are 2,4-D-ammonium, 2,4-D-dimethylammonium, 2,4-D-diethylammonium, 2,4-D-diethanolammonium (2,4-D-diolamine), 2,4-D-triethanolammonium, 2,4-D-isopropylammonium, 2,4-D-triisopropanolammonium, 2,4-D-heptylammonium, 2,4-D-dodecylammonium, 2,4-D-tetradecylammonium, 2,4-D-triethylammonium, 2,4-D-tris(2-hydroxypropyl)ammonium, 2,4-D-tris(isopropyl)ammonium, 2,4-D-trolamine, 2,4-D-lithium, 2,4-D-sodium and 2,4-D-N,N,N-trimethylethanolammonium (2,4-D choline). Examples of suitable esters of 2,4-D are 2,4-D-butotyl, 2,4-D-2-butoxypropyl, 2,4-D-3-butoxypropyl, 2,4-D-butyl, 2,4-D-ethyl, 2,4-D-ethylhexyl, 2,4-D-isobutyl, 2,4-D-isooctyl, 2,4-D-isopropyl, 2,4-D-meptyl, 2,4-D-methyl, 2,4-D-octyl, 2,4-D-pentyl, 2,4-D-propyl, 2,4-D-tefuryl and clacyfos.
Suitable salts of 2,4-DB are for example 2,4-DB-sodium, 2,4-DB-potassium and 2,4-DB-dimethylammonium. Suitable esters of 2,4-DB are for example 2,4-DB-butyl and 2,4-DB-isoctyl. Suitable salts of dichlorprop are for example dichlorprop-sodium, dichlorprop-potassium and dichlorprop-dimethylammonium. Examples of suitable esters of dichlorprop are dichlorprop-butotyl and dichlorprop-isooctyl.
Suitable salts and esters of MCPA include MCPA-butotyl, MCPA-butyl, MCPA-dimethylammonium, MCPA-diolamine, MCPA-ethyl, MCPA-thioethyl, MCPA-2-ethylhexyl, MCPA-isobutyl, MCPA-isooctyl, MCPA-isopropyl, MCPA-isopropylammonium, MCPA-methyl, MCPA-olamine, MCPA-potassium, MCPA-sodium and MCPA-trolamine.
A suitable salt of MCPB is MCPB sodium. A suitable ester of MCPB is MCPB-ethyl.
Suitable salts of clopyralid are clopyralid-potassium, clopyralid-olamine and clopyralid-tris-(2-hydroxypropyl)ammonium. Example of suitable esters of clopyralid is clopyralid-methyl. Examples of a suitable ester of fluroxypyr are fluroxypyr-meptyl and fluroxypyr-2-butoxy-1-methylethyl, wherein fluroxypyr-meptyl is preferred.
Suitable salts of picloram are picloram-dimethylammonium, picloram-potassium, picloram-triisopropanolammonium, picloram-triisopropylammonium and picloram-trolamine. A suitable ester of picloram is picloram-isoctyl.
A suitable salt of triclopyr is triclopyr-triethylammonium. Suitable esters of triclopyr are for example triclopyr-ethyl and triclopyr-butotyl.
Suitable salts and esters of chloramben include chloramben-ammonium, chloramben-diolamine, chloramben-methyl, chloramben-methylammonium and chloramben-sodium. Suitable salts and esters of 2,3,6-TBA include 2,3,6-TBA-dimethylammonium, 2,3,6-TBA-lithium, 2,3,6-TBA-potassium and 2,3,6-TBA-sodium.
Suitable salts and esters of aminopyralid include aminopyralid-potassium, aminopyralid-dimethylammonium, and aminopyralid-tris(2-hydroxypropyl)ammonium.
Suitable salts and esters of bromoxynil are for example bromoxynil-butyrate, bromoxynil-heptanoate, bromoxynil-octanoate, bromoxynil-potassium and bromoxynil-sodium.
Suitable salts and esters of ioxonil are for example ioxonil-octanoate, ioxonil-potassium and ioxonil-sodium.
Suitable salts and esters of mecoprop include mecoprop-butotyl, mecoprop-dimethylammonium, mecoprop-diolamine, mecoprop-ethadyl, mecoprop-2-ethylhexyl, mecoprop-isoctyl, mecopropmethyl, mecoprop-potassium, mecoprop-sodium and mecoprop-trolamine.
Suitable salts of mecoprop-P are for example mecoprop-P-butotyl, mecoprop-P-dimethylammonium, mecoprop-P-2-ethylhexyl, mecoprop-P-isobutyl, mecoprop-P-potassium and mecoprop-P-sodium.
A suitable salt of diflufenzopyr is for example diflufenzopyr-sodium.
A suitable salt of naptalam is for example naptalam-sodium.
Suitable salts and esters of aminocyclopyrachlor are for example aminocyclopyrachlor-dimethylammonium, aminocyclopyrachlor-methyl, aminocyclopyrachlor-triisopropanolammonium, aminocyclopyrachlor-sodium and aminocyclopyrachlor-potassium.
A suitable salt of quinclorac is for example quinclorac-dimethylammonium.
A suitable salt of quinmerac is for example quinmerac-dimethylammonium.
A suitable salt of imazamox is for example imazamox-ammonium.
Suitable salts of imazapic are for example imazapic-ammonium and imazapic-isopropylammonium.
Suitable salts of imazapyr are for example imazapyr-ammonium and imazapyr-isopropylammonium.
A suitable salt of imazaquin is for example imazaquin-ammonium.
Suitable salts of imazethapyr are for example imazethapyr-ammonium and imazethapyr-isopropylammonium.
A suitable salt of topramezone is for example topramezone-sodium.

According to a preferred embodiment of the invention, the composition comprises as herbicidal active compound B or component B at least one, preferably exactly one herbicide B.

According to another preferred embodiment of the invention, the composition comprises as herbicidal active compounds B or component B at least two, preferably exactly two herbicides B different from each other.

According to another preferred embodiment of the invention, the composition comprises as herbicidal active compounds B or component B at least three, preferably exactly three herbicides B different from each other.

According to another preferred embodiment of the invention, the composition comprises as safening component C or component C at least one, preferably exactly one safener C.

According to another preferred embodiment of the invention, the composition comprises as component B at least one, preferably exactly one herbicide B, and as component C at least one, preferably exactly one, safener C.

According to another preferred embodiment of the invention, the composition comprises at least two, preferably exactly two, herbicides B different from each other, and as component C at least one, preferably exactly one, safener C.

According to another preferred embodiment of the invention, the composition comprises at least three, preferably exactly three, herbicides B different from each other, and as component C at least one, preferably exactly one, safener C.

According to another preferred embodiment of the invention, the composition comprises, at least one and especially exactly one herbicidally active compound from group b1), in particular selected from the group consisting of clethodim, clodinafop-propargyl, cycloxydim, cyhalofop-butyl, fenoxaprop-ethyl, fenoxaprop-P-ethyl, metamifop, pinoxaden, profoxydim, sethoxydim, tepraloxydim, tralkoxydim, esprocarb, ethofumesate, molinate, prosulfocarb, thiobencarb and triallate.

According to another preferred embodiment of the invention, the composition comprises, at least one and especially exactly one herbicidally active compound from group b2), in particular selected from the group consisting of bensulfuron-methyl, bispyribac-sodium, cloransulam-methyl, chlorsulfuron, clorimuron, cyclosulfamuron, diclosulam, florasulam, flumetsulam, flupyrsulfuron-methyl-sodium, foramsulfuron, imazamox, imazamox-ammonium, imazapic, imazapic-ammonium, imazapic-isopropylammonium, imazapyr, imazapyr-ammonium, imazethapyr-isopropylammonium, imazaquin, imazaquin-ammonium, imazethapyr, imazethapyr-ammonium, imazethapyr-isopropylammonium, imazosulfuron, iodosulfuron-methyl-sodium, iofensulfuron, iofensulfuron-sodium, mesosulfuron-methyl, metazosulfuron, metsulfuron-methyl, metosulam, nicosulfuron, penoxsulam, propoxycarbazon-sodium, pyrazosulfuron-ethyl, pyribenzoxim, pyriftalid, pyroxsulam, propyrisulfuron, rimsulfuron, sulfosulfuron, thiencarbazon-methyl, thifensulfuron-methyl, tribenuron-methyl, tritosulfuron and triafamone.

According to another preferred embodiment of the invention, the composition comprises, at least one and especially exactly one herbicidally active compound from group b3), in particular selected from the group consisting of ametryn, atrazine, bentazon, bromoxynil, bromoxynil-octanoate, bromoxynil-heptanoate, bromoxynil-potassium, diuron, fluometuron, hexazinone, isoproturon, linuron, metamitron, metribuzin, paraquat-dichloride, propanil, simazin, terbutryn and terbuthylazine.

According to another preferred embodiment of the invention, the composition comprises at least one and especially exactly one herbicidally active compound from group b4), in particular selected from the group consisting of acifluorfen, butafencil, carfenetrazone-ethyl, flumioxazin, fomesafen, oxadiargyl, oxyfluorfen, pyraflufen, pyraflufen-ethyl, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)-phenoxy]-2-pyridyloxy]acetate (CAS 353292-31-6; S-3100)..

According to another preferred embodiment of the invention, the composition comprises, at least one and especially exactly one herbicidally active compound from group b5), in particular selected from the group consisting of amitrole, benzobicyclon, bicyclopyrone, clomazone, diflufenican, fenquintrone, fluometuron, flurochloridone, isoxaflutole, mesotrione, norflurazone, oxotrione (CAS 1486617-21-3), picolinafen, sulcotrione, tefuryltrione, tembotrione, tolpyralate, topramezone, topramezone-sodium, 2-chloro-3-methylsulfanyl-N-(1-methyltetrazol-5-yl)-4-(trifluoromethyl)benzamide (CAS 1361139-71-0), bixlozone , 2-(2,5-dichlorophenyl)methyl-4,4-dimethyl-3-isoxazolidinone (CAS 81778-66-7), and chloro-2-[-3-(difluoromethyl)isoxazol-5-yl]phenyl-5-chloropyrimidin-2-yl-ether.

According to another preferred embodiment of the invention, the composition comprises, at least one and especially exactly one herbicidally active compound from group b9), in particular selected from the group consisting of pendimethalin and trifluralin.

According to another preferred embodiment of the invention, the composition comprises at least one and especially exactly one herbicidally active compound from group b10), in particular selected from the group consisting of acetochlor, butachlor, cafenstrole, dimethenamid-P, fentrazamide, flufenacet, mefenacet, metazachlor, metolachlor, S-metolachlor, fenoxasulfone, ipfencarbazone and pyroxasulfone.
Likewise, preference is given to compositions comprising at least one and especially exactly one herbicidally active compound from group b10), in particular selected from the group consisting of isoxazoline compounds of the formulae II.1, II.2, II.3, II.4, II.5, II.6, II.7, II.8 and II.9, as defined above.

According to another preferred embodiment of the invention, the composition comprises, at least one and especially exactly one herbicidally active compound from group b11), in particular indaziflam, isoxaben and triaziflam.

According to another preferred embodiment of the invention, the composition comprises, at least one and especially exactly one herbicidally active compound from group b13), in particular selected from the group consisting of 2,4-D, 2,4-D-isobutyl, 2,4-D-dimethylammonium, 2,4-D-N,N,N-trimethylethanolammonium, aminocyclopyrachlor, aminocyclopyrachlor-potassium, aminocyclopyrachlor-methyl, aminopyralid, aminopyralid-methyl, aminopyralid-dimethylammonium, aminopyralid-tris(2-hydroxypropyl)ammonium, clopyralid, clopyralid-methyl, clopyralid-olamine, flopyrauxifen, fluroxypyr, fluroxypyr-meptyl, halauxifen, halauxifen-methyl, MCPA, MCPA-2-ethylhexyl, MCPA-dimethylammonium, quinclorac, quinclorac-dimethylammonium, quinmerac, quinmerac-dimethylammonium, florpyrauxifen, florpyrauxifen-benzyl (CAS 1390661-72-9), and 4-amino-3-chloro-5-fluoro-6-(7-fluoro-1H-indol-6-yl)picolinic acid (CAS 1629965-65-6).

According to another preferred embodiment of the invention, the composition comprises, at least one and especially exactly one herbicidally active compound from group b14), in particular selected from the group consisting of diflufenzopyr, diflufenzopyr-sodium, dymron, indanofan and diflufenzopyr-sodium.

According to another preferred embodiment of the invention, the composition comprises at least one and especially exactly one herbicidally active compound from group b15), in particular selected from the group consisting of cinmethylin, dymron (= daimuron), indanofan, oxaziclomefone and tetflupyrolimet.

According to another preferred embodiment of the invention, the composition comprises at least one and especially exactly one safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

In binary compositions two active compounds, the weight ratio these active compounds is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising a herbicidal active substance B and at least one safener C, the weight ratio of the herbicidal active substance B : safener C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising two herbicidal active compounds B and at least one safener C, the relative proportions by weight of the herbicidal components B are generally in the range of from 1:1000 to 1000:1, preferably in the range of from 1:500 to 500:1, in particular in the range of from 1:250 to 250:1 and particularly preferably in the range of from 1:75 to 75:1, the weight ratio of each herbicide B : components C is generally in the range of from 1:1000 to 1000:1, preferably in the range of from 1:500 to 500:1, in particular in the range of from 1:250 to 250:1 and particularly preferably in the range of from 1:75 to 75:1, and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1..

Particularly preferred herbicides B are the herbicides B as defined above; in particular the herbicides B.1 - B.214 listed below in table B:

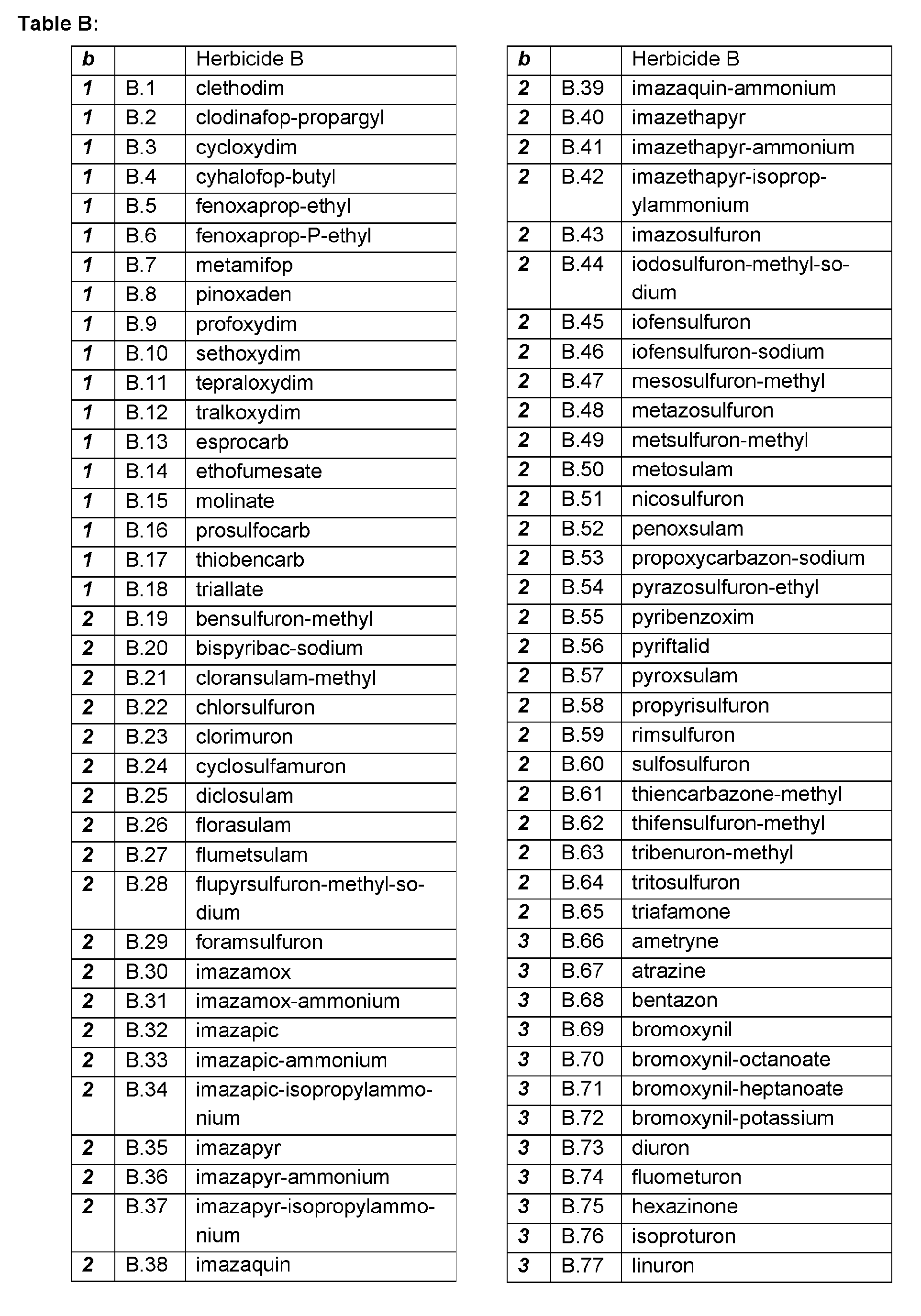

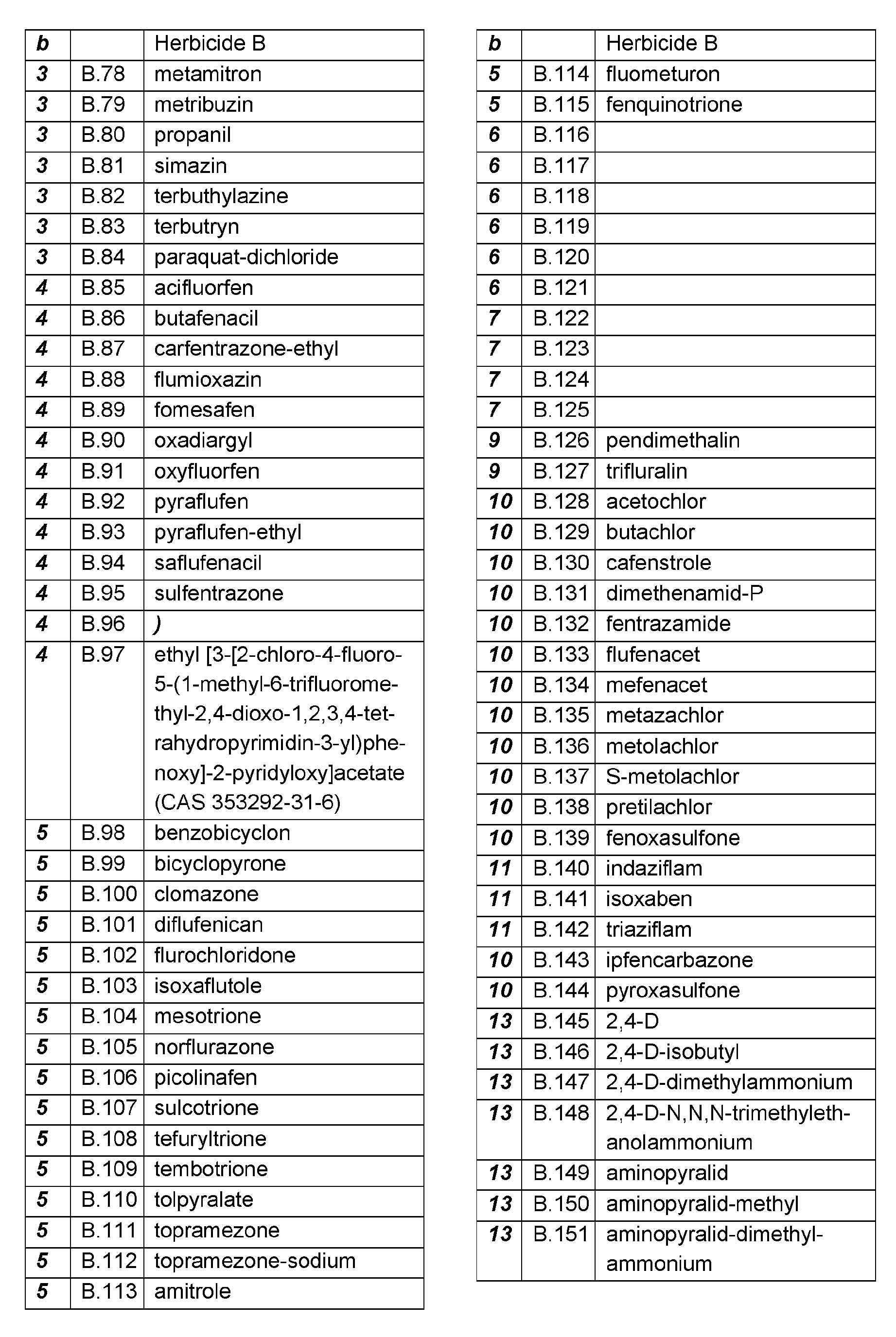

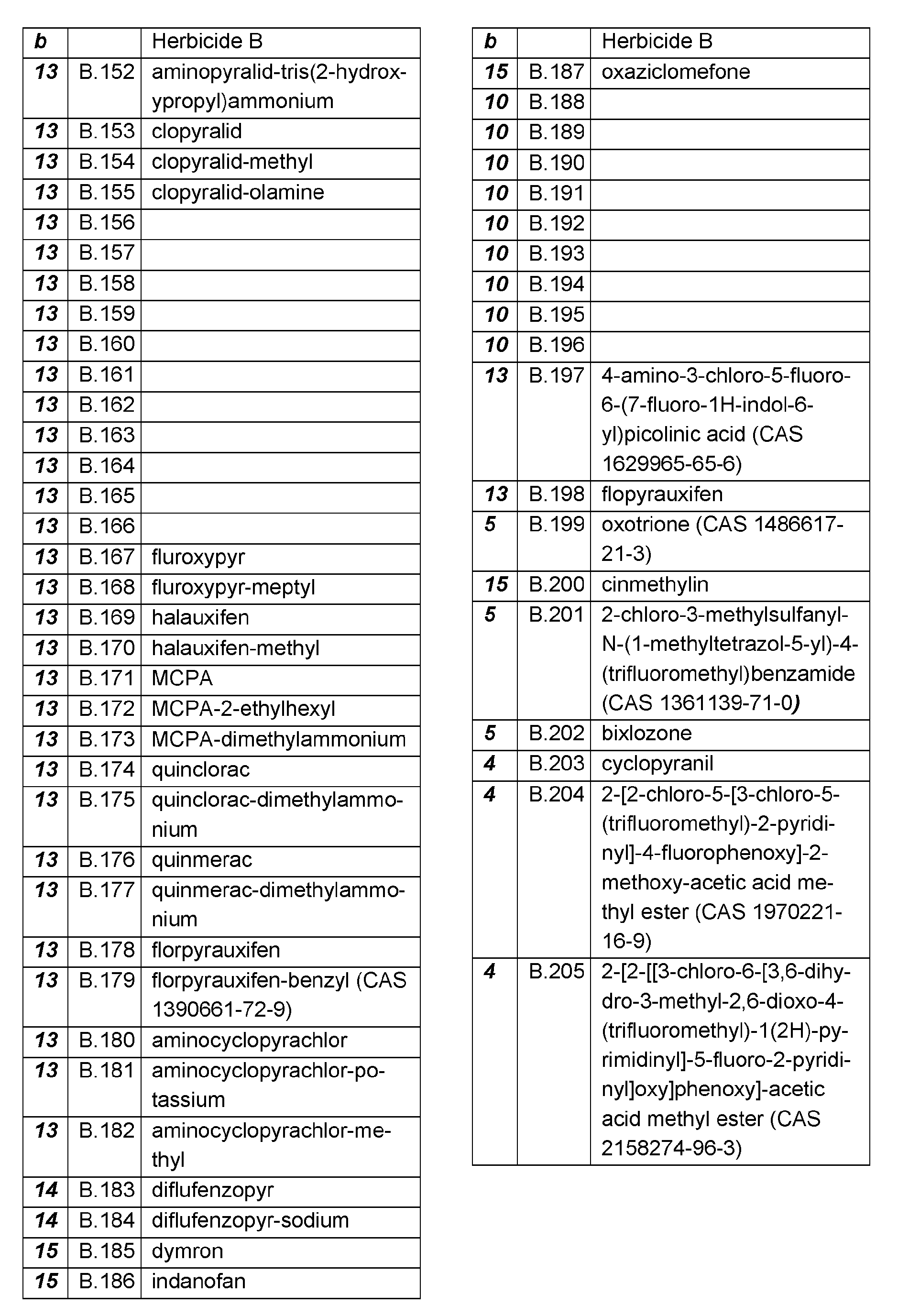

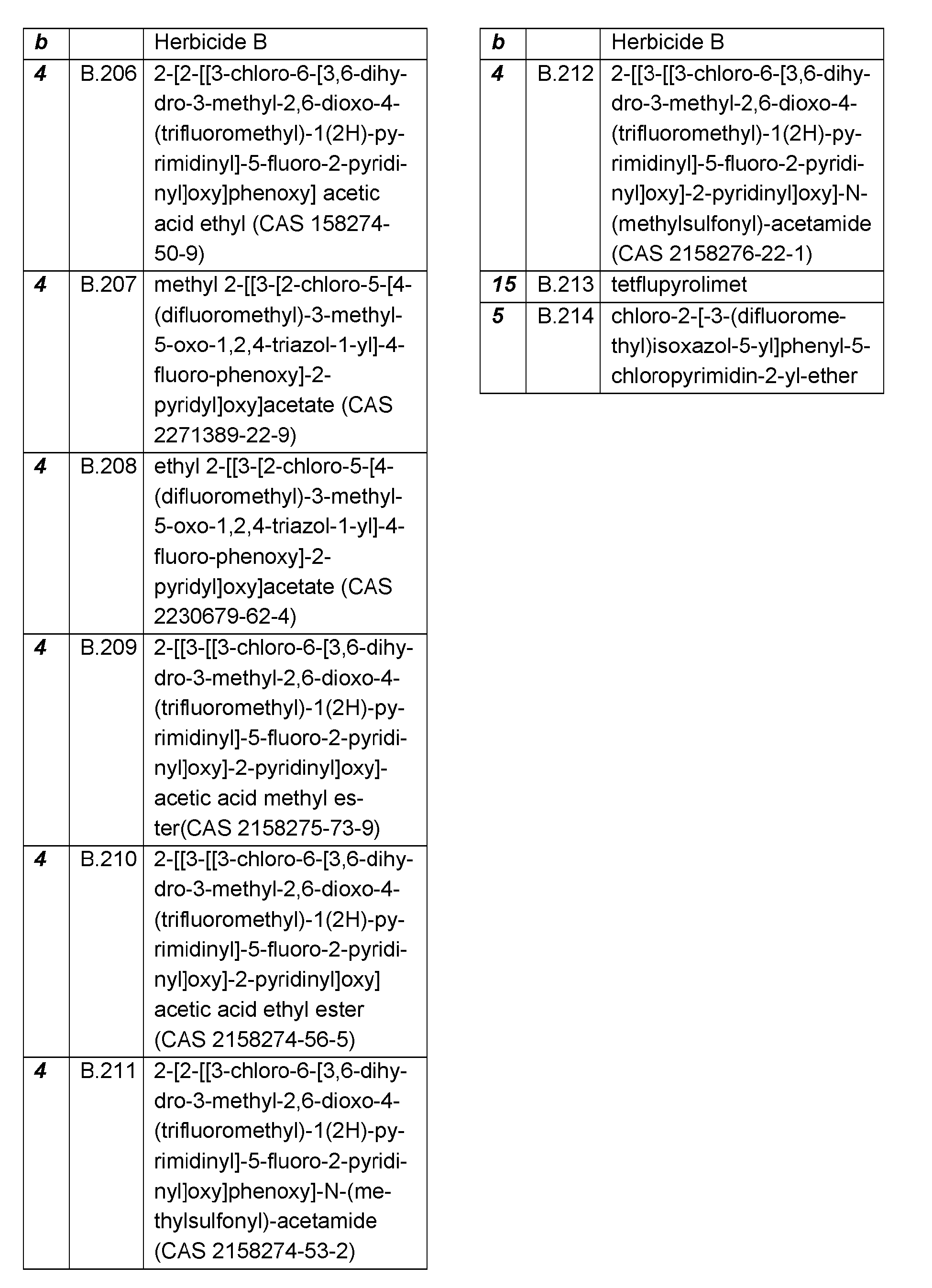

Particularly preferred safeners C, which, as component C, are constituent of the composition according to the invention are the safeners C as defined above; in particular the safeners C.1 - C.17 listed below in table C:

**Table C**

| | Safener C |
|---|---|
| C.1 | benoxacor |
| C.2 | cloquintocet |
| C.3 | cloquintocet-mexyl |
| C.4 | cyprosulfamide |
| C.5 | dichlormid |
| C.6 | fenchlorazole |
| C.7 | fenchlorazole-ethyl |
| C.8 | fenclorim |
| C.9 | furilazole |
| C.10 | isoxadifen |
| C.11 | isoxadifen-ethyl |
| C.12 | mefenpyr |
| C.13 | mefenpyr-diethyl |
| C.14 | naphtalic acid anhydride |
| C.15 | 4-(dichloroacetyl)-1-oxa-4-azaspiro[4.5]decane (MON4660, CAS 71526-07-3) |
| C.16 | 2,2,5-trimethyl-3-(dichloroacetyl)-1,3-oxazolidine (R-29148, CAS 52836-31-4) |
| C.17 | metcamifen |

The weight ratios of the individual components in the preferred mixtures mentioned below are within the limits given above, in particular within the preferred limits.

Preferred herbicides as the active substance are the following or their mixtures:
Atrazine, a triazine herbicide used in corn and sorghum for control of broadleaf weeds and grasses. It is still used because of its low cost and because it works as a synergist when used with other herbicides, it is a photosystem II inhibitor.
Clopyralid, a broadleaf herbicide in the pyridine group, used mainly in turf, rangeland, and for control of noxious thistles. Notorious for its ability to persist in compost. It is another example of synthetic auxin.
Imazapyr, a non-selective herbicide used for the control of a broad range of weeds including terrestrial annual and perennial grasses and broadleaved herbs, woody species, and riparian and emergent aquatic species.
Imazapic, a selective herbicide for both the pre- and post-emergent control of some annual and perennial grasses and some broadleaf weeds. Imazapic kills plants by inhibiting the production of branched chain amino acids (valine, leucine, and isoleucine), which are necessary for protein synthesis and cell growth.
Metoalachlor, a pre-emergent herbicide widely used for control of annual grasses in corn and sorghum; it has largely replaced atrazine for these uses.
Paraquat, a nonselective contact herbicide used for no-till burn down and in aerial destruction of marijuana and coca plantings. More acutely toxic to people than any other herbicide in widespread commercial use.
Picloram, a pyridine herbicide mainly used to control unwanted trees in pastures and edges of fields. It is another synthetic auxin.
Triclopyr.
Cinmethylin
Saflufenacil
trifludimoxazin

In one embodiment, the active substance is a herbicide selected from cinmethylin, dimethenamid-P, clomazone, picolinafen, metazachlor, S.metalochlor, acetochlor, pendimethalin, saflufenacil, pyroxasulfone, bixlozone, prosulfocarb, flufenacet, Aclonifen, Triallate, Flumioxazin, Metazachlor, Pethoxamid, Napropamid, Terbuthylazin, Isoxaflutole, Isoxaben, Metamitron, trifludimoxazin, Tiafenacil, Sulfentrazone or mixtures thereof.

In one embodiment, the active substance is a herbicide selected from cinmethylin, dimethenamid-P, clomazone, picolinafen, metazachlor, S-metalochlor, acetochlor, pendimethalin, saflufenacil, pyroxasulfone, bixlozone, or mixtures thereof.

In one embodiment, the active substance is a mixture of dimethenamid-P and clomazone.
In one embodiment, the active substance is a mixture of cinmethylin and picolinafen.
In one embodiment, the active substance is cinmethylin.

Insecticides: An insecticide is a pesticide used against insects in all developmental forms. They include ovicides and larvicides used against the eggs and larvae of insects. Insecticides are used in agriculture, medicine, industry and the household. In the following, suitable insecticides are mentioned:
O) Insecticides from classes O.1 to O.28
O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
O.2 GABA-gated chloride channel antagonists: endosulfan, chlordane; ethiprole, fipronil, flufiprole, pyrafluprole, pyriprole;
O.3 Sodium channel modulators: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; DDT, methoxychlor;
O.4 Nicotinic acetylcholine receptor (nAChR) agonists: acetamiprid, clothianidin, cycloxaprid, dinotefuran, imidacloprid, nitenpyram, thiacloprid, thiamethoxam; 4,5-dihydro-*N*-nitro-1-(2-oxiranylmethyl)-1*H*-imidazol-2-amine, (2*E*)-1-[(6-chloropyridin-3-yl)methyl]-*N*'-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine; sulfoxaflor, flupyradifurone, triflumezopyrim, (3*R*)-3-(2-chlorothiazol-5-yl)-8-methyl-5-oxo-6-phenyl-2,3-dihydrothiazolo[3,2-a]pyrimidin-8-ium-7-olate, (3*S*)-3-(6-chloro-3-pyridyl)-8-methyl-5-oxo-6-phenyl-2,3-dihydrothiazolo[3,2-a]pyrimidin-8-ium-7-olate, (3*S*)-8-methyl-5-oxo-6-phenyl-3-pyrimidin-5-yl-2,3-dihydrothiazolo[3,2-a]pyrimidin-8-ium-7-olate, (3*R*)-3-(2-chlorothiazol-5-yl)-8-methyl-5-oxo-6-[3-(trifluoromethyl)phenyl]-2,3-dihydrothiazolo[3,2-a]pyrimidin-8-ium-7-olate; (3*R*)-3-(2-chlorothiazol-5-yl)-6-(3,5-dichlorophenyl)-8-methyl-5-oxo-2,3-dihydrothiazolo[3,2-a]pyrimidin-8-ium-7-olate, (3*R*)-3-(2-chlorothiazol-5-yl)-8-ethyl-5-oxo-6-phenyl-2,3-dihydrothiazolo[3,2-a]pyrimidin-8-ium-7-olate;
O.5 Nicotinic acetylcholine receptor allosteric activators: spinosad, spinetoram;
O.6 Chloride channel activators: abamectin, emamectin benzoate, ivermectin, lepimectin, milbemectin;
O.7 Juvenile hormone mimics: hydroprene, kinoprene, methoprene; fenoxycarb, pyriproxyfen;
O.8 miscellaneous non-specific (multi-site) inhibitors: methyl bromide and other alkyl halides; chloropicrin, sulfuryl fluoride, borax, tartar emetic;
O.9 Chordotonal organ TRPV channel modulators: pymetrozine, pyrifluquinazon;
O.10 Mite growth inhibitors: clofentezine, hexythiazox, diflovidazin; etoxazole;
O.11 Microbial disruptors of insect midgut membranes: *Bacillus thuringiensis, Bacillus sphaericus* and the insecticdal proteins they produce: *Bacillus thuringiensis* subsp. *israelensis, Bacillus sphaericus, Bacillus thuringiensis* subsp. *aizawai, Bacillus thuringiensis* subsp. *kurstaki, Bacillus thuringiensis* subsp. *tenebrionis,* the Bt crop proteins: Cry1Ab, Cry1Ac, Cry1Fa, Cry2Ab, mCry3A, Cry3Ab, Cry3Bb, Cry34/35Ab1;
0.12 Inhibitors of mitochondrial ATP synthase: diafenthiuron; azocyclotin, cyhexatin, fenbutatin oxide, propargite, tetradifon;
0.13 Uncouplers of oxidative phosphorylation via disruption of the proton gradient: chlorfenapyr, DNOC, sulfluramid;
0.14 Nicotinic acetylcholine receptor (nAChR) channel blockers: bensultap, cartap hydrochloride, thiocyclam, thiosultap sodium;
0.15 Inhibitors of the chitin biosynthesis type 0: bistrifluron, chlorfluazuron, diflubenzuron, flucycloxuron, flufenoxuron, hexaflumuron, lufenuron, novaluron, noviflumuron, teflubenzuron, triflumuron;
0.16 Inhibitors of the chitin biosynthesis type 1: buprofezin;
0.17 Moulting disruptors: cyromazine;
0.18 Ecdyson receptor agonists: methoxyfenozide, tebufenozide, halofenozide, fufenozide, chromafenozide;
0.19 Octopamin receptor agonists: amitraz;
O.20 Mitochondrial complex III electron transport inhibitors: hydramethylnon, acequinocyl, fluacrypyrim, bifenazate;
0.21 Mitochondrial complex I electron transport inhibitors: fenazaquin, fenpyroximate, pyrimidifen, pyridaben, tebufenpyrad, tolfenpyrad; rotenone;
O.22 Voltage-dependent sodium channel blockers: indoxacarb, metaflumizone, 2-[2-(4-cyanophenyl)-1-[3-(trifluoromethyl)phenyl]ethylidene]-*N*-[4-(difluoromethoxy)phenyl]-hydrazinecarboxamide, *N*-(3-chloro-2-methylphenyl)-2-[(4-chlorophenyl)-[4-[methyl(methylsulfonyl)amino]phenyl]methylene]-hydrazinecarboxamide;
O.23 Inhibitors of the of acetyl CoA carboxylase: spirodiclofen, spiromesifen, spirotetramat, spiropidion;
O.24 Mitochondrial complex IV electron transport inhibitors: aluminium phosphide, calcium phosphide, phosphine, zinc phosphide, cyanide;
O.25 Mitochondrial complex II electron transport inhibitors: cyenopyrafen, cyflumetofen;
O.26 Ryanodine receptor-modulators: flubendiamide, chlorantraniliprole, cyantraniliprole, cyclaniliprole, tetraniliprole; (*R*)-3-chloro-*N*¹-{2-methyl-4-[1,2,2,2 -tetrafluoro-1-(trifluoromethyl)-ethyl]phenyl}-*N*²-(1-methyl-2-methylsulfonylethyl)phthalamide, (*S*)-3-chloro-*N*¹-{2-methyl-4-[1,2,2,2-tetrafluoro-1-(trifluoromethyl)ethyl]phenyl}-*N*²-(1-methyl-2-methylsulfonylethyl)-phthalamide, methyl-2-[3,5-dibromo-2-(([3-bromo-1-(3-chloropyridin-2-yl)-1*H*-pyrazol-5-yl]carbonyl}amino)benzoyl]-1,2-dimethylhydrazinecarboxylate; *N*-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; *N*-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; *N*-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; *N*-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; *N*-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide; 3-chloro-1-(3-chloro-2-pyridinyl)-*N*-[2,4-dichloro-6-[[(1-cyano-1-methylethyl)amino]carbonyl]phenyl]-1*H*-pyrazole-5-carboxamide; tetrachlorantraniliprole; *N*-[4-chloro-2-[[(1,1-dimethylethyl)amino]carbonyl]-6-methylphenyl]-1-(3-chloro-2-pyridinyl)-3-(fluoromethoxy)-1*H*-pyrazole-5-carboxamide; cyhalodiamide;
O.27: Chordotonal organ modulators - undefined target site: flonicamid;
O.28. insecticidal compounds of unknown or uncertain mode of action: afidopyropen, afoxolaner, azadirachtin, amidoflumet, benzoximate, broflanilide, bromopropylate, chinomethionat, cryolite, dicloromezotiaz, dicofol, flufenerim, flometoquin, fluensulfone, fluhexafon, fluopyram, fluralaner, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1*H*-1,2,4-triazole-5-amine, *Bacillus firmus* I-1582; flupyrimin; fluazaindolizine; 4-[5-(3,5-dichlorophenyl)-5-(trifluoromethyl)-4*H-*isoxazol-3-yl]-2-methyl-*N*-(1-oxothietan-3-yl)benzamide; fluxametamide; 5-[3-[2,6-dichloro-4-(3,3-dichloroallyloxy)phenoxy]propoxy]-1*H*-pyrazole; 4-cyano-*N*-[2-cyano-5-[[2,6-dibromo-4-[1,2,2,3,3,3-hexafluoro-1-(trifluoromethyl)propyl]phenyl]carbamoyl]phenyl]-2-methyl-benzamide; 4-cyano-3-[(4-cyano-2-methyl-benzoyl)amino]-*N*-[2,6-dichloro-4-[1,2,2,3,3,3-hexafluoro-1-(trifluoromethyl)propyl]phenyl]-2-fluoro-benzamide; *N*-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; *N*-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; *N*-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; 4-cyano-*N*-[2-cyano-5-[[2,6-dichloro-4-[1,2,2,3,3,3-hexafluoro-1-(trifluoromethyl)propyl]phenyl]carbamoyl]phenyl]-2-methyl-benzamide; 4-cyano-*N*-[2-cyano-5-[[2,6-dichloro-4-[1,2,2,2-tetrafluoro-1-(trifluoromethyl)ethyl]phenyl]carbamoyl]phenyl]-2-methyl-benzamide; *N*-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; 2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; *N*-methylsulfonyl-6-[2-(3-pyridyl)thiazol-5-yl]pyridine-2-carboxamide; *N*-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-isopropyl-*N*,5-dimethyl-*N*-pyridazin-4-yl-pyrazole-4-carboxamide; 1-(1,2-dimethylpropyl)-*N*-ethyl-5-methyl-*N*-pyridazin-4-yl-pyrazole-4-carboxamide; *N*,5-dimethyl-*N*-pyridazin-4-yl-1-(2,2,2-trifluoro-1-methyl-ethyl)pyrazole-4-carboxamide; 1-[1-(1-cyanocyclopropyl)ethyl]-*N*-ethyl-5-methyl-*N*-pyridazin-4-yl-pyrazole-4-carboxamide; *N*-ethyl-1-(2-fluoro-1-methyl-propyl)-5-meth-yl-*N*-pyridazin-4-yl-pyrazole-4-carboxamide; 1-(1,2-dimethylpropyl)-*N*,5-dimethyl-*N*-pyridazin-4-yl-pyrazole-4-carboxamide; 1-[1-(1-cyanocyclopropyl)ethyl]-*N*,5-dimethyl-*N*-pyridazin-4-yl-pyrazole-4-carboxamide; *N*-methyl-1-(2-fluoro-1-methyl-propyl]-5-methyl-*N*-pyridazin-4-yl-pyrazole-4-carboxamide; 1-(4,4-difluorocyclohexyl)-*N*-ethyl-5-methyl-*N*-pyridazin-4-yl-pyrazole-4-carboxamide; 1-(4,4-difluorocyclohexyl)-*N*,5-dimethyl-*N*-pyridazin-4-yl-pyrazole-4-carboxamide, *N*-(1-methylethyl)-2-(3-pyridinyl)-2*H*-indazole-4-carboxamide; *N*-cyclopropyl-2-(3-pyridinyl)-2*H*-indazole-4-carboxamide; *N*-cyclohexyl-2-(3-pyridinyl)-2*H*-indazole-4-carboxamide; 2-(3-pyridinyl)-*N*-(2,2,2-trifluoroethyl)-2*H*-indazole-4-carboxamide; 2-(3-pyridinyl)-*N*-[(tetrahydro-2-furanyl)methyl]-2*H-*indazole-5-carboxamide; methyl 2-[[2-(3-pyridinyl)-2*H*-indazol-5-yl]carbonyl]hydrazinecarboxylate; *N*-[(2,2-difluorocyclopropyl)methyl]-2-(3-pyridinyl)-2*H*-indazole-5-carboxamide; *N*-(2,2-difluoropropyl)-2-(3-pyridinyl)-2*H*-indazole-5-carboxamide; 2-(3-pyridinyl)-*N*-(2-pyrimidinylmethyl )-2*H*-indazole-5-carboxamide; *N*-[(5-methyl-2-pyrazinyl)methyl]-2-(3-pyridinyl)-2*H*-indazole-5-carboxamide, tyclopyrazoflor; sarolaner, lotilaner, *N*-[4-chloro-3-[[(phenylmethyl)amino]carbonyl]phenyl]-1-methyl-3-(1,1,2,2,2-pentafluoroethyl)-4-(trifluoromethyl)-1*H*-pyrazole-5-carboxamide; 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine, isocycloseram, *N*-[4-chloro-3-(cyclopropylcarbamoyl)phenyl]-2-methyl-5-(1,1,2,2,2-pentafluoroethyl)-4-(trifluoromethyl)pyrazole-3-carboxamide, *N*-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; acynonapyr; benzpyrimoxan; tigolaner; chloro-*N*-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide, oxazosulfyl, [(2*S*,3*R*,4*R*,5*S*,6*S*)-3,5-dimethoxy-6-methyl-4-propoxy-tetrahydropyran-2-yl]-*N*-[4-[1-[4-(trifluoromethoxy)phenyl]-1,2,4-triazol-3-yl]phenyl]carbamate, [(2*S*,3*R*,4*R*,5*S*,6*S*)-3,4,5-trimethoxy-6-methyl-tetrahydropyran-2-yl] N-[4-[1-[4-(trifluoromethoxy)phenyl]-1,2,4-triazol-3-yl]phenyl]carbamate, [(2*S*,3*R*,4*R*,5*S*,6*S*)-3,5-dimethoxy-6-methyl-4-propoxy-tetrahydropyran-2-yl]-*N*-[4-[1-[4-(1,1,2,2,2-pentafluoroethoxy)phenyl]-1,2,4-triazol-3-yl]phenyl]carbamate, [(2*S,*3*R*,4*R*,5*S*,6*S*)-3,4,5-trimethoxy-6-methyl-tetrahydropyran-2-yl]-*N*-[4-[1-[4-(1,1,2,2,2-pentafluoroethoxy)phenyl]-1,2,4-triazol-3-yl]phenyl]carbamate, (2*Z*)-3-(2-isopropylphenyl)-2-[(*E*)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-[3-ethylsulfonyl-6-(trifluoromethyl)imidazo[1,2-a]pyridin-2-yl]-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine,
   pyrethrum extract, icaridin, N,N-Diethyl-meta-toluamide (DEET), p-menthane diole (PMD), metofluthrin, meperfluthrin, dimeflluthrin, permethrin, cypermethrin, deltamethrin, heptafluthrin, d-heptafluthrin, tetramethrin, imiprothrin, saturated and/or unsaturated fatty acids, d-tetramethrin, d-phenothrin, 1R trans phenothrin, transfluthrin, d-allethrin, d-trans allethrin 75/25, prallethrin, piperonyl butoxide and its analogues/homologues, and their mixtures.

Preferred insecticides are the following:
∘ Chlorinated insecticides such as, for example, Camphechlor, Hexachlorocyclohexane, gamma-Hexachlorocyclohexane, Methoxychlor, Pentachlorophenol, TDE, Aldrin, Chlordane, Chlordecone, Dieldrin, Endosulfan, Endrin, Heptachlor, Mirex and their mixtures;
∘ Organophosphorus compounds such as, for example, Acephate, Azinphos-methyl, Ben¬sulide, Chlorethoxyfos, Chlorpyrifos, Chlorpyriphos-methyl, Diazinon, Dichlorvos (DDVP), Dicrotophos, Dimethoate, Disulfoton, Ethoprop, Fenamiphos, Fenitrothion, Fenthion, Fosthiazate, Malathion, Methamidophos, Methidathion, Methyl-parathion, Mevinphos, Naled, Omethoate, Oxydemeton-methyl, Parathion, Phorate, Phosalone, Phosmet, Phostebupirim, Pirimiphos-methyl, Profenofos, Terbufos, Tetrachlorvinphos, Tribufos, Trichlorfon and their mixture;
∘ Pyrethroids such as, for example, Allethrin, Bifenthrin, Deltamethrin, Permethrin, Resmethrin, Sumithrin, Tetramethrin, Tralomethrin, Transfluthrin and their mixtures;
∘ Plant toxin derived compounds such as, for example, Derris (rotenone), Pyrethrum, Neem (Azadirachtin), Nicotine, Caffeine and their mixtures.
   Rodenticides: Rodenticides are a category of pest control chemicals intended to kill rodents. In the following, examples for suitable rodenticides are given:
∘ Anticoagulants, e.g. difenacoum, brodifacoum, floccumafen, bromadiolone, difethialone, warfarin, coumatetralyl, chlorophacinone, diphacinone, coumachlor, coumafuryl and pindone;
∘ Metal phosphides;
∘ Phosphides; or
∘ Hypercalcemia, e.g. Calciferols (vitamins D), cholecalciferol (vitamin D3) and ergocalciferol (vitamin D2).

Miticides, moluscicides and nematicides: Miticides are pesticides that kill mites. Antibiotic miticides, carbamate miticides, formamidine miticides, mite growth regulators, organochlorine, permethrin and organophosphate miticides all belong to this category. Molluscicides are pesticides used to control mollusks, such as moths, slugs and snails. These substances include metaldehyde, methiocarb and aluminium sulfate. A nematicide is a type of chemical pesticide used to kill parasitic nematodes (a phylum of worm). A nematicide is obtained from a neem tree's seed cake; which is the residue of neem seeds after oil extraction. The neem tree is known by several names in the world but was first cultivated in India since ancient times.

The pesticide usually has a water-solubility up to 10 g/l, preferably up to 5 g/l, and more preferably up to 1 g/l.

The pesticide may be solid or liquid at 20 °C.

Preferred pesticides are herbicides, insecticides and fungicides.
In one embodiment, the active substance is a herbicide.
In one embodiment, the active substance is a fungicide.
In one embodiment, the active substance is an insecticide.

In one embodiment, the active substance is a mixture of dimethenamid-P and clomazone.
In one embodiment, the active substance is a mixture of cinmethylin and picolinafen.

Especially preferred pesticides as the active substance are tepraloxydim, flufenacet, napropamid, isoxaben, fluazifop-P-butyl, metamitron, propyzamide, phenmedipham, clethodim, chloridazon, dimethenamid-P, pendimethalin, Chlorpyrifos, dimethoate alpha-cypermethrin, cypermethrin, clothianidin, chlorfenapyr, fipronil, dimethenamid-P, clomazone, picolinafen, metazachlor, S-metalochlor, acetochlor, pendimethalin, saflufenacil, pyroxasulfone, bixlozone, pyraclostrobin, dimethenamide-P, fenpropimorph, saflufenacil, trifludimoxazin and cinmethylin .

In one embodiment, the active substance is a herbicide selected from dimethenamid-P, clomazone, picolinafen, metazachlor, S.metalochlor, acetochlor, pendimethalin, saflufenacil, pyroxasulfone, bixlozone, cinmethylin or mixtures thereof.

Particularly preferred pesticides as the active substance are cinmethylin, pyraclostrobin, dimethenamide-P.

In one embodiment, the active substance is selected from pyrethrum extract, icaridin, N,N-Diethyl-meta-toluamide (DEET), p-menthane diole (PMD), metofluthrin, meperfluthrin, dimeflluthrin, permethrin, cypermethrin, deltamethrin, heptafluthrin, d-heptafluthrin, tetramethrin, imiprothrin, saturated and/or unsaturated fatty acids, d-tetramethrin, d-phenothrin, 1R trans phenothrin, transfluthrin, d-allethrin, d-trans allethrin 75/25, prallethrin, piperonyl butoxide and its analogues/homologues, essential oils and their components, and their mixtures.

In one embodiment, the active substance is selected from pyrethrum extract, icaridin, N,N-Diethyl-meta-toluamide (DEET), p-menthane diole (PMD), metofluthrin, meperfluthrin, dimeflluthrin, permethrin, cypermethrin, deltamethrin, heptafluthrin, d-heptafluthrin, tetramethrin, imiprothrin, saturated and/or unsaturated fatty acids, d-tetramethrin, d-phenothrin, 1R trans phenothrin, transfluthrin, d-allethrin, d-trans allethrin 75/25, prallethrin, piperonyl butoxide and its analogues/homologues, and their mixtures.

Typically, microparticles of the invention contain from 1 to 95 wt%, preferably 10 to 90 wt%, more preferably 30 to 85 wt% of said one or more active substance.

In principle, active substance may be a liquid or a solid at 21 °C, where the solid itself may also be present dissolved in a water immiscible solvent S.

In one embodiment, the active substance used in the capsule according to the invention is liquid at 21°C.
In one embodiment, the active substance used in the capsule according to the invention is liquid at 21°C and is contained in the microparticles of the invention without being dissolved in a solvent.
In one embodiment, the active substance used in the capsule according to the invention is a liquid at 21°C and is contained in the microparticles of the invention as a pure substance.

In one embodiment, the active substance is comprised in microparticles of the invention as a solution in a water immiscible solvent S.
Sometimes, the active substance can also act as a solvent, or a solvent can also act as an active substance.

Water immiscible solvents S have a solubility in water of 1 wt% or less at 21 °C, preferably of 0.1 wt% or less at 21 °C.

Solvents S include:
mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes and C8 to C11 aromatic petroleum derivatives (aromatic hydrocarbons) with a boiling point range from 130°C to 300°C;
vegetable oils such as coco oil, palm kern oil, palm oil, soya oil, rapeseed oil, corn oil and the methyl or ethyl esters of the afore-mentioned oils, hydrocarbons such as aromatic depleted, linear paraffinic, isoparaffinic, cycloparaffinic having a flash point between 40°C and 250°C and a distillation range between 150°C and 450°C;
ketones, e.g. acetophenone;
carbonates, e.g. dibutyl carbonate;
esters, e.g. benzyl acetate, methyl benzoate, ethyl benzoate, propyl benzoate, butyl benzoate, benzyl lactate, 2-phenoxyethyl propionate;
lactates, e.g. 2-ethylhexyl lactate;
fatty acid esters,
fatty acids;
phosphonates;
fatty acid amines;
pyrrolidones, such as N-octylpyrrolidone, N-ethyl pyrrolidone, N-docedyl pyrrolidone;
fatty acid amides, e.g. N,N-dimethyloctanamide, N,N-dimethylnonaneamide, N,N-dimethyldecanamide, N,N-Dimethyl 9-decenamide, lauryl N,N-dimethylamide, lauryl N,N-dimethylamide, and mixtures thereof.

Herein, "C8 dimethylamide" and "N,N-dimethyl octaneamide" shall be understood to mean "C8 fatty acid N,N-dimethylamide" (analogously for other chain lengths).

"Fatty acid" herein shall denote a linear or branched carboxylic acid with a saturated or unsaturated aliphatic chain.

In one embodiment, solvent S is an oil. The phrase "oil" in the context of the present invention encompasses all kinds of oil bodies or oil components, in particular vegetable oils like e.g. rape seed oil, sunflower oil, soy oil, olive oil and the like, modified vegetable oils e.g. alkoxylated sunflower or soy oil, synthetic (tri)glycerides like e.g. technical mixtures of mono, di and triglycerides of C6-C22 fatty acids, fatty acid alkyl esters e.g. methyl or ethyl esters of vegetable oils (Agnique^{®} ME 18 RD-F, Agnique^{®} ME 18 SD-F, Agnique^{®} ME 12C-F, Agnique^{®} ME1270, all products of BASF SE, Germany) fatty acid alkyl esters based on said C6-C22 fatty acids, mineral oils and their mixtures. In one form the oil comprises preferably mineral oils.

Examples illustrating the nature of suitable solvents S without limiting the invention to these examples are: Guerbet alcohols based on fatty alcohols having 6 to 18, preferably 8 to 10, carbon atoms, esters of linear C6-C22-fatty acids with linear or branched C6-C22-fatty alcohols or esters of branched C6-C 13-carboxylic acids with linear or branched C6-C 22-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6-C22-fatty acids with branched alcohols, in particular 2-ethylhexanol, esters of C18-C38- alkyl hydroxy carboxylic acids with linear or branched C6-C 22-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6-C10-fatty acids, liquid mono-/di-/triglyceride mixtures based on C6-C18-fatty acids, esters of C6-C22-fatty alcohols and/or Guerbet alcohols with aromatic carboxylic acids, in particular benzoic acid, esters of C2- C12-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C6-C22-fatty alcohol carbonates, such as, for example, dicaprylyl carbonate (Cetiol^{®} CC), Guerbet carbonates, based on fatty alcohols having 6 to 18, preferably 8 to 10, carbon atoms, esters of benzoic acid with linear and/or branched C6-C22-alcohols, linear or branched, symmetrical or asymmetrical dialkyl ethers having 6 to 22 carbon atoms per alkyl group, such as, for example, dicaprylyl ether, ring-opening products of epoxidized fatty acid esters with polyols, silicone oils (cyclomethicones, silicone methicone grades, etc.), aliphatic or naphthenic hydrocarbons, such as, for example, squalane, squalene or dialkylcyclohexanes, and/or mineral oils. In one form the oil comprises preferably aliphatic or naphthenic hydrocarbons, and/or mineral oils.

Within the context of the present invention, preferred solvents S are, Guerbet alcohols based on fatty alcohols having 6 to 18, preferably 8 to 10, carbon atoms, esters of linear C6-C22-fatty acids with linear or branched C6-C22-fatty alcohols or esters of branched C6-C13-carboxylic acids with linear or branched C6-C22-fatty alcohols, such as e.g. myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate.

Also preferred oils are esters of linear C6-C22-fatty acids with branched alcohols, in particular 2-ethylhexanol, esters of C18-C38-alkylhydroxycarboxylic acids with linear or branched C6-C22-fatty alcohols, linear or branched C6-C22-fatty alcohols, in particular dioctyl malates, esters of linear and/or branched fatty acids with polyhydric alcohols (such as e.g. propylene glycol, dimerdiol or trimertriol) and/or Guerbet alcohols, triglycerides based on C6-C10-fatty acids, liquid mono-/di-/triglyceride mixtures based on C6-C18-fatty acids, esters of C6-C22-fatty alcohols and/or Guerbet alcohols with aromatic carboxylic acids, in particular benzoic acid, esters of C2-C12-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C6-C22-fatty alcohol carbonates, such as e.g. dicaprylyl carbonate (Cetiol^{™} CC), Guerbet carbonates based on fatty alcohols having 6 to 18, preferably 8 to 10, carbon atoms, esters of benzoic acid with linear and/or branched C6-C22-alcohols (e.g. Finsolv^{™} TN), linear or branched, symmetrical or asymmetrical dialkyl ethers having 6 to 22 carbon atoms per alkyl group, such as e.g. dicaprylyl ether (Cetiol^{™} OE), ring-opening products of epoxidized fatty acid esters with polyols, silicone oils (cyclomethicones, silicon methicone types etc.) and/or aliphatic or naphthenic hydrocarbons, such as e.g. squalane, squalene or dialkylcyclohexanes.

Furthermore, liquid linear and/or branched and/or saturated or unsaturated hydrocarbons or any desired mixtures thereof can be used as oils within the context of the present invention. These may be e.g. alkanes having 4 to 22, preferably 6 to 18, carbon atoms, or any desired mixtures thereof. Also of suitability are the unsaturated hydrocarbons having 4 to 22 carbon atoms, or unsaturated hydrocarbons of identical carbon number, and any desired mixtures of these hydrocarbons. Cyclic hydrocarbons and aromatics, e.g. toluene and mixtures thereof may also be oils within the context of the present invention. In another preferred form the oil comprises aromatics. Also suitable are silicone oils. Any desired mixtures of all of the specified core materials

Customary oil components in cosmetics are, for example, paraffin oil, glyceryl stearate, isopropyl myristate, diisopropyl adipate, dibutyl adipate, cetylstearyl 2-ethylhexanoate, hydrogenated polyisobutene, vaseline, caprylic/capric triglycerides, microcrystalline wax, lanolin and stearic acid. However, this list is exemplary and not exhaustive.

Particular preference is given to those sparingly water-soluble or water-insoluble organic active substances which are soluble or suspendable in the water-insoluble or sparingly water-soluble sol-gel precursor which is used for constructing the shell of the capsule according to the invention.

Preferred solvents S, especially for pesticides as active substances, are:
mineral oil fractions of medium to high boiling point, e.g. kerosene, diesel oil; oils of vegetable or
animal origin; aliphatic, cyclic and aromatic hydrocarbons, e. g. toluene, paraffin, tetrahydronaphthalene, alkylated naphthalenes and C8 to C11 aromatic petroleum derivatives (aromatic hydrocarbons) with a boiling point range from 130°C to 300°C;
vegetable oils such as coco oil, palm kern oil, palm oil, soya oil, rapeseed oil, corn oil and the methyl or ethyl esters of the afore-mentioned oils, hydrocarbons such as aromatic depleted, linear paraffinic, isoparaffinic, cycloparaffinic having a flash point between 40°C and 250°C and a distillation range between 150°C and 450°C;
acetophenone; dibutyl carbonate; benzyl acetate, methyl benzoate, ethyl benzoate, propyl benzoate, butyl benzoate, benzyl lactate, 2-phenoxyethyl propionate; 2-Ethylhexyl lactate; fatty acid esters; fatty acids; C8-C12 fatty acid dimethyl amides; and mixtures thereof.

More preferred organic solvents S are:
acetophenone; dibutyl carbonate; benzyl acetate, methyl benzoate, ethyl benzoate, propyl benzoate, butyl benzoate, benzyl lactate, 2-phenoxyethyl propionate; 2-Ethylhexyl lactate; fatty acid esters; fatty acids; C8-C12 fatty acid dimethyl amides; and mixtures thereof.

C8-C12 fatty acid dimethyl amides include and preferred C8-C12 fatty acid dimethyl amides are: C8 dimethyl amide (N,N-dimethyloctanamide), C8/C10 dimethyl amide (mixture of N,N-dimethyloctanamide and N,N-dimethyldecanamide), C9 dimethyl amide (N,N-dimethylnonaneamide or N,N-Dimethyl iso-nonaneamide), C10 dimethyl amide (N-Dimethyldecanamide or N,N-Dimethyl 9-decenamide), C12 dimethyl amide (lauryl N,N-dimethylamide), vegetable oils such as coco oil, palm kern oil, palm oil, soya oil, rapeseed oil, corn oil and the methyl or ethyl esters of the afore-mentioned oils.

Especially preferred organic solvents S are vegetable oils such as coco oil, palm kern oil, palm oil, soya oil, rapeseed oil, corn oil and the methyl or ethyl esters of the afore-mentioned oils, benzylacetate, methylbenzoate, C8-C12 fatty acid dimethyl amide, aromatic hydrocarbon or their mixtures.

Particularly preferred organic solvents S are, aromatic hydrocarbon, adipates (e.g. dibutyladipate), vegetable oils such as coco oil, palm kern oil, palm oil, soya oil, rapeseed oil, corn oil and the methyl or ethyl esters of the afore-mentioned oils or their mixtures.

In one embodiment, form the core of microparticles or the inventions contains a pesticide blended with a solvent S selected from aliphatic and/or aromatic hydrocarbons or vegetable oils such as coco oil, palm kern oil, palm oil, soya oil, rapeseed oil, corn oil and the methyl or ethyl esters of the afore-mentioned oil.

Besides the one or more active substance and optionally solvent S, microparticles of the invention, in particular the microspheres or the core of the microcapsules according to the invention, can also comprise **auxiliaries** which are usually used in the respective fields of application.

Microparticles according to the invention contain a matrix material or a shell that surrounds the core.
Said matrix material or shell comprises
i) at least one phospholipid PL, and
ii) optionally at least one sterol ST.

Microparticles of the invention are **biomimetic,** meaning that they comprise a matrix material or a shell containing naturally occurring phospholipids and optionally sterols or derivatives of such naturally occurring phospholipids and sterols and optionally minerals or inorganic salts.
Typically, microparticles of the invention are free from microplastics and materials that form microplastics.
In one embodiment, microparticles of the invention are vegan, meaning that its components do not originate from or were obtained using any animals.
Phospholipids, sometimes also referred to as phosphatides, are a class of lipids that is generally known to the skilled person and whose molecular structure contains a hydrophilic "head" containing a phosphate group, and two hydrophobic "tails" derived from fatty acids and/or fatty alcohols, linked by a polyalcohol residue (e.g. glycerol) or aminoalcohol. The phosphate group can be modified with simple polyfunctional organic molecules such as choline, ethanolamine or serine or sugars (e.g. Inositol).
Phospholipids wherein the hydrophobic part is are least partly derived from fatty alcohols are also referred to a phospholipid ethers or plasmologens.
Preferably, phospholipids contain two hydrophobic "tails" that are esters of fatty acids with a polyalcohol residue (e.g. glycerol) or an aminoalcohol. Preferred phospholipids contain two hydrophobic "tails" that are esters of fatty acids with glycerol.
Phospholipids are amphiphilic.

The term "lipid" refers to biomolecules that have a high solubility in unpolar solvents such as hydrocarbons.

The term "phospholipids" as used herein includes naturally occurring phospholipids as well as synthetic phospholipids.

In one embodiment, phospholipid PL is selected from Glycerophospholipids (also referred to as phosphoglycerides) and Phosphosphingolipids, with Glycerophospholipids being preferred.

Preferred phospholipids PL are Phosphatidic acids (phosphatidates), Phosphatidylethanolamines (cephalin), Phosphatidylcholines (lecithin (e.g. egg yolk lecithin, asolectin and soybean lecithin), Phosphatidylserines, Phosphoinositides, Phosphatidylinositols, Phosphatidylinositol phosphates, bisphosphates, Phosphatidylinositols, Ceramide phosphorylcholines (Sphingomyelin), Ceramide phosphorylethanolamines (Sphingomyelin), Ceramide phosphoryllipids or mixtures thereof.
Asolectin is a preferred phospholipid PL and is mixture of phospholipids obtained commercially from soybeans that contains lecithin, cephalin and inositol phosphatides.

Natural phospholipids are typically purified from, e.g., soybeans, sunflower or egg yolk, for example using solvent extraction and chromatographic procedures. Preferred sources of phospholipids are soybean and sunflower. Synthetic phospholipids with specific polar head group, fatty acid composition can be manufactured using various synthesis routes. They can be synthesized de novo, or naturally occurring phospholipids can be derivatized, e.g. by hydrogenation of double bonds or by enzymatic derivatization.

Examples of derivatives of phospholipids include
Phosphatidic acid (DMPA, DPPA, DSPA),
Phosphatidylcholine (DDPC, DLPC, DMPC, DPPC, DSPC, DOPC, POPC, DEPC),
Phosphatidylglycerol (DMPG, DPPG, DSPG, POPG),
Phosphatidylethanolamine (DMPE, DPPE, DSPE DOPE),
Phosphatidylserine (DOPS) and
PEG phospholipid (mPEG-phospholipid, polyglycerin-phospholipid, functionalized-phospholipid, terminal activated-phospholipid).

Synthetic phospholipids with the natural stereochemical configuration are for example synthesized from glycerophosphocholine (GPC), which is obtained from natural phospholipids, using acylation and enzyme catalyzed reactions.

In especially preferred embodiments, phospholipid PL is asolectin, lecithin or a mixture thereof. In one embodiment, phospholipid PL is obtained from soybean, rapeseed, sunflower, eggs of birds (e.g. chicken eggs), bovine milk or fish eggs.
In one embodiment, phospholipid PL obtained from soybean, rapeseed or sunflower.
In one embodiment, phospholipid PL obtained from soybean or sunflower.
In one embodiment, phospholipid PL is lecithin obtained from soybean or sunflower.

Specific examples of phospholipids include the following:

| Abbreviation | CAS | Name | Type |
|---|---|---|---|
| DDPC | 3436-44-0 | 1,2-Didecanoyl-sn-glycero-3-phosphocholine | Phosphatidylcholine |
| DEPA-NA | 80724-31-8 | 1,2-Dierucoyl-sn-glycero-3-phosphate (Sodium Salt) | Phosphatidic acid |
| DEPC | 56649-39-9 | 1,2-Dierucoyl-sn-glycero-3-phosphocholine | Phosphatidylcholine |
| DEPE | 988-07-2 | 1,2-Dierucoyl-sn-glycero-3-phosphoethanolamine | Phosphatidylethanolamine |
| DEPG-NA | | 1,2-Dierucoyl-sn-glycero-3[Phospho-rac-(1-glycerol...) (Sodium Salt) | Phosphatidylglycerol |
| DLOPC | 998-06-1 | 1,2-Dilinoleoyl-sn-glycero-3-phosphocholine | Phosphatidylcholine |
| DLPA-NA | | 1,2-Dilauroyl-sn-glycero-3-phosphate (Sodium Salt) | Phosphatidic acid |
| DLPC | 18194-25-7 | 1,2-Dilauroyl-sn-glycero-3-phosphocholine | Phosphatidylcholine |
| DLPE | | 1,2-Dilauroyl-sn-glycero-3-phosphoethanolamine | Phosphatidylethanolamine |
| DLPG-NA | | 1,2-Dilauroyl-sn-glycero-3[Phospho-rac-(1-glycerol...) (Sodium Salt) | Phosphatidylglycerol |
| DLPG-NH4 | | 1,2-Dilauroyl-sn-glycero-3[Phospho-rac-(1-glycerol...) (Ammonium Salt) | Phosphatidylglycerol |
| DLPS-NA | | 1,2-Dilauroyl-sn-glycero-3-phosphoserine (Sodium Salt) | Phosphatidylserine |
| DMPA-NA | 80724-3 | 1,2-Dimyristoyl-sn-glycero-3-phosphate (Sodium Salt) | Phosphatidic acid |
| DMPC | 18194-24-6 | 1,2-Dimyristoyl-sn-g lycero-3-phosphocholine | Phosphatidylcholine |
| DMPE | 988-07-2 | 1,2-Dimyristoyl-sn-g lycero-3-phosphoethanolamine | Phosphatidylethanolamine |
| DMPG-NA | 67232-80-8 | 1,2-Dimyristoyl-sn-glycero-3[Phospho-rac-(1-glycerol...) (Sodium Salt) | Phosphatidylglycerol |
| DMPG-NH4 | | 1,2-Dimyristoyl-sn-glycero-3[Phospho-rac-(1-glycerol...) (Ammonium Salt) | Phosphatidylglycerol |
| DMPG-NH4/NA | | 1,2-Dimyristoyl-sn-glycero-3[Phospho-rac-(1-glycerol...) (Sodium/Ammonium Salt) | Phosphatidylglycerol |
| DMPS-NA | | 1,2-Dimyristoyl-sn-glycero-3-phosphoserine (Sodium Salt) | Phosphatidylserine |
| DOPA-NA | | 1,2-Dioleoyl-sn-glycero-3-phosphate (Sodium Salt) | Phosphatidic acid |
| DOPC | 4235-95-4 | 1,2-Dioleoyl-sn-glycero-3-phosphocholine | Phosphatidylcholine |
| DOPE | 4004-5-1- | 1,2-Dioleoyl-sn-glycero-3-phosphoethanolamine | Phosphatidylethanolamine |
| DOPG-NA | 62700-69-0 | 1,2-Dioleoyl-sn-glycero-3[Phospho-rac-(1-glycerol...) (Sodium Salt) | Phosphatidylglycerol |
| DOPS-NA | 70614-14-1 | 1,2-Dioleoyl-sn-glycero-3-phosphoserine (Sodium Salt) | Phosphatidylserine |
| DPPA-NA | 71065-87-7 | 1 ,2-Dipalmitoyl-sn-glycero-3-phosphate (Sodium Salt) | Phosphatidic acid |
| DPPC | 63-89-8 | 1,2-Dipalmitoyl-sn-glycero-3-phosphocholine | Phosphatidylcholine |
| DPPE | 923-61-5 | 1,2-Dipalmitoyl-sn-glycero-3-phosphoethanolamine | Phosphatidylethanolamine |
| DPPG-NA | 67232-81-9 | 1,2-Dipalmitoyl-sn-glycero-3[Phospho-rac-(1-glycerol...) (Sodium Salt) | Phosphatidylglycerol |
| DPPG-NH4 | 73548-70-6 | 1,2-Dipalmitoyl-sn-glycero-3[Phospho-rac-(1-glycerol...) (Ammonium Salt) | Phosphatidylglycerol |
| DPPS-NA | | 1,2-Dipalmitoyl-sn-glycero-3-phosphoserine (Sodium Salt) | Phosphatidylserine |
| DSPA-NA | 108321-18-2 | 1,2-Distearoyl-sn-glycero-3-phosphate (Sodium Salt) | Phosphatidic acid |
| DSPC | 816-94-4 | 1,2-Distearoyl-sn-glycero-3-phosphocholine | Phosphatidylcholine |
| DSPE | 1069-79-0 | 1,2-Distearoyl-sn-glycero-3-phosphoethanolamine | Phosphatidylethanolamine |
| DSPG-NA | 67232-82-0 | 1,2-Distearoyl-sn-glycero-3[Phospho-rac-(1-glycerol...) (Sodium Salt) | Phosphatidylglycerol |
| DSPG-NH4 | 108347-80-4 | 1,2-Distearoyl-sn-glycero-3[Phospho-rac-(1-glycerol...) (Ammonium Salt) | Phosphatidylglycerol |
| DSPS-NA | | 1,2-Distearoyl-sn-glycero-3-phosphoserine (Sodium Salt) | Phosphatidylserine |
| EPC | | Egg-PC | Phosphatidylcholine |
| HEPC | | Hydrogenated Egg PC | Phosphatidylcholine |
| HSPC | | Hydrogenated Soy PC | Phosphatidylcholine |
| LYSOPC MYRISTIC | 18194-24-6 | 1-Myristoyl-sn-glycero-3-phosphocholine | Lysophosphatidylcholine |
| LYSOPC PALMITIC | 17364-16-8 | 1-Palmitoyl-sn-glycero-3-phosphocholine | Lysophosphatidylcholine |
| LYSOPC STEARIC | 19420-57-6 | 1-Stearoyl-sn-glycero-3-phosphocholine | Lysophosphatidylcholine |
| Milk Sphingomyelin MPPC | | 1-Myristoyl-2-palmitoyl-sn-glycero 3-phosphocholine | Phosphatidylcholine |
| MSPC | | 1-Myristoyl-2-stearoyl-sn-glycero-3-phosphocholine | Phosphatidylcholine |
| PMPC | | 1-Palmitoyl-2-myristoyl-sn-glycero-3-phosphocholine | Phosphatidylcholine |
| POPC | 26853-31-6 | 1-Palmitoyl-2-oleoyl-sn-glycero-3-phosphocholine | Phosphatidylcholine |
| POPE | | 1-Palmitoyl-2-oleoyl-sn-glycero-3-phosphoethanolamine | Phosphatidylethanolamine |
| POPG-NA | 81490-05-3 | 1-Palmitoyl-2-oleoyl-sn-glycero-3[Phospho-rac-(1-glycerol)...] (Sodium Salt) | Phosphatidylglycerol |
| PSPC | | 1-Palmitoyl-2-stearoyl-sn-glycero-3-phosphocholine | Phosphatidylcholine |
| SMPC | | 1-Stearoyl-2-myristoyl-sn-glycero-3-phosphocholine | Phosphatidylcholine |
| SOPC | | 1-Stearoyl-2-oleoyl-sn-glycero-3-phosphocholine | Phosphatidylcholine |
| SPPC | | 1-Stearoyl-2-palmitoyl-sn-glycero-3-phosphocholine | Phosphatidylcholine |

The microparticles of the invention, in particular the microspheres or the shell of microcapsules of the invention, optionally further comprise at least one **sterol ST.**
In one embodiment, the microparticles of the invention, in particular the microspheres or the shell of microcapsules of the invention, further comprises at least one **sterol ST.**
Sterols are chemical compounds containing a 3-hydroxy gonane backbone.

Sterol ST can be a phytosterol, a zoosterol or a synthetic sterol.

In one embodiment, Sterol ST is a zoosterol.
In one embodiment, Sterol ST is a phytosterol.
In one embodiment, Sterol ST is a synthetic sterol.
In one embodiment, Sterol ST is a synthetic sterol that does naturally occur.

In on embodiment, sterol ST is prepared by extraction of plants and contains a mixture of different sterols. Whenever reference is made herein to a certain sterol suitable as sterol ST, this shall include mixtures of such sterol with other sterols.

In one embodiment, sterol ST is selected from cholesterol, beta sitosterol, beta sitostanol, stigmasterol, stigmastanol, campesterol, campestanol, ergosterol, avenasterol, brassicasterol, lanosterol, soy sterols, wood sterols, rape sterols or mixtures thereof.

In one embodiment, sterol ST is selected from cholesterol, beta sitosterol, ergosterol, lanosterol, soy sterols, wood sterols, rape sterols, or mixtures thereof.
In one embodiment, sterol ST is selected from beta sitosterol, lanosterol, soy sterols, wood sterols, rape sterols or mixtures thereof.

In one preferred embodiment, the mass ratio of phospholipid PL (component i) to sterol ST (component ii) in the microparticles is from 1:10 to 10:1.

In one embodiment, microparticles of the invention further comprise an inorganic salt or a mineral, said mineral having a solubility in water of less than 0.01 wt% at 21°C.

Typically, said inorganic salt or mineral is present in the form of solid particles having an average particle diameter d50 that is smaller than the particle size of the microparticles wherein said particles of inorganic salt or mineral form a coating on the surface of said microparticle.

In one preferred embodiment, said inorganic salt or mineral is a phosphate containing inorganic salt or mineral.

Preferably, said inorganic salt or a mineral is selected from hydroxy apatite, tricalcium phosphate, calcium hydrogenophosphates.

Typically said inorganic salt or a mineral is added to the formulation such that the ratio of phospholipid PL to inorganic salt or a mineral is from 1:2 to 50 :1.

In one embodiment, microparticles of the invention contain a nonionic **surfactant.**
Typically, a nonionic surfactant is present at the interface of the capsule shell and the water shell. It is also possible that certain amounts of surfactants are present in the core of the capsule and the water phase.

Suitable nonionic surfactants include alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Examples of nonionic surfactants include the neutral surface-active compounds of the formula (II),

R'-(O-B)ₙ-OH (II)

wherein
R' is a hydrocarbon residue having from 8 to 40 and more preferably from 12 to 30 carbon atoms and optionally one oxygen atom,
B is C₂-C₄-alkane-1,2-diyl, such as 1,2-ethylene, 1,2-propylene or 1,2-butylene or a combination thereof and more preferred 1,2-ethylene or a combination thereof with 1,2-propylene, and
n is from 3 to 100, preferably from 4 to 50 and more preferred from 5 to 40.

Preferred nonionic surfactants include block copolymers of ethylene oxide (EO) and propylene oxide (PO). Such block copolymers can for example have the structure R-(EO)x-(PO)y-(EO)z, with R being H or a C₄ to C₃₀ alkyl rest and x, y, z independently being numbers from 2 to 100.

Examples of suitable hydrocarbon R' include the residue mentioned for R. In a preferred embodiment of the invention the residue R' is a phenyl residue being substituted with one C4-C18-alkyl group.

Further preferred examples or nonionic surfactants are ethoxylates of sorbate molecules. Preferred are ethoxylates of polysorbates that bear terminal ester groups with fatty acids, such as C₆ to C₃₀, especially C₁₂ to C₁₈ fatty acids.

Typically, if present, formulations containing microparticles of the invention contain 0.01 to 5 wt%, preferably 0.1 to 1 wt% or 0.1 to 0,.5 wt% of nonionic surfactants, based on the formulation.
Typically, if present, microparticles of the invention contain 0.01 to 5 wt%, preferably 0.05 to 0.5 wt% of nonionic surfactant, based on the microparticles.

The shape the microparticles according to the invention is typically spherical or essentially spherical.

Microparticles of the invention typically have an average diameter d50 of 0.1 to 20 µm, preferably 0.5 to 10 µm. All particle sizes given herein are determined by statistic laser scattering using a Malvern Mastersizer 2000 according to European norm ISO 13320 EN.

Another aspect of the invention is directed to processes for making microparticles, comprising the following steps:
A) Providing a non-aqueous mixture containing one or more active substance, at least one phospholipid PL, optionally at least one sterol ST and optionally a nonaqueous solvent that is not miscible with water, wherein phospholipid PL and said sterol ST are at least partially dissolved in said nonaqueous solvent or said one or more pesticides,
B) Emulsifying the non-aqueous mixture obtained in step A) with water, optionally supported by stirring and/or surfactants,
C) Optionally adding at least one inorganic salt or mineral, preferably phosphate containing salt or mineral, having a solubility in water of less than 0.1 wt% at 21°.

By adding at least one inorganic salt or mineral in step C), the microparticles obtained in step B) are be coated with particles of such inorganic salt or mineral.

Typically, microparticles prepared according to processes of the invention are microcapsules having a shell and a core or are microspheres.

In one embodiment, step B) is carried out such that an oil in water emulsion is obtained in step B).

In one embodiment, the reaction medium in step A) is acidic.

In one embodiment, said inorganic salt is added in step C) such that the mixture obtained comprises 0.001 to 5 wt%, more preferably 0.002 to 1wt %, especially preferably 0.005 to 0.1 wt% of said inorganic salt, based on the entire mixture.

In one embodiment, the surfactant used in step B) is a nonionic surfactant.

It was of the surprising results of the invention that the process for making microparticles of the invention, including the encapsulation step and the crosslinking using inorganic salts, especially phosphate salts, can be carried out at room temperature or without the need for cooling the reaction mixture.

Another aspect of the invention are microparticles obtainable by processes according to the invention as described above and with the embodiments as described.
Another aspect of the invention are microparticles obtained by processes according to the invention as described above and with the embodiments as described.

Another aspect of the invention are formulations comprising microparticles of the invention or microparticles prepared according to processes of the invention.

Microparticles of the invention or microparticles prepared according to processes of the invention can be converted into customary types of agrochemical compositions suspensions, pastes, granules, pressings or mixtures thereof.

In one embodiment, microparticles containing formulations of the invention are liquid formulations, wherein the microparticles are present as dispersed particles in solvent (i.e. a suspension), preferably an aqueous medium.

The term "aqueous medium" stands for the liquid phase of the composition and comprises an aqueous solvent and optionally compounds dissolved therein, e.g. surfactants as mentioned above, and if present, conventional one or more conventional formulation additives, such as thickeners or biocides. The aqueous solvent of the aqueous suspension is either water or a mixture thereof with a water-miscible organic solvent, such as C1-C4-alkanols, e.g. methanol, ethanol, n-propanol, isopropanol, n-butanol, 2-butanol, isobutanol, or tert. butanol, C2-C5-alkanediols and C3-C8- alkanetriols, preferably from the group consisting of ethylene glycol, 1,2-propanediol, 1,3-propanediol, glycerol and 1,4-butanediol. Generally, the amount of water in the aqueous solvent is at least 50% by weight, in particular at least 80% by weight or at least 90 % by weight, based on the aqueous solvent. The aqueous solvent may consist mainly of water, i.e. water makes up at least 95% by weight of the total amount of solvent present in the suspension. The aqueous solvent may also be a mixture of the aforementioned water-miscible organic solvent and water. In the latter case, the weight ratio of water to water-miscible organic solvent in the aqueous solvent preferably is in the range of from 99:1 to 1:1; more preferably in the range of from 50:1 to 3:1; and most preferably in the range of from 20:1 to 4:1. Expressed differently the amount of organic solvent may be from 1 to 50% by weight, more preferably from 2 to 25% by weight, and most preferably from 5 to 20% by weight, based on the total weight of the aqueous solvent.

Formulations of the invention may comprise one or more further active substances outside the microparticles. Such further active substances can for example be dissolved in the solvent medium, preferably the aqueous phase, or may be present as solid particles that are dispersed in the solvent medium, preferably the aqueous phase.

In one embodiment, formulations of the invention comprise 1 to 50 wt%, preferably 5 to 45 wt%, more preferably 10 to 40 wt% of said one or more active substances based on the formulation.

If present, the concentration of surfactants in the aqueous suspension will frequently be in the range from 0.01 to 10% by weight, in particular from 0.05 to 5% by weight, based on the total weight of the aqueous suspension of the microparticles.

The aqueous compositions according to the invention may also comprise customary formulation auxiliaries. Examples of auxiliaries include such as viscosity-modifying additives (thickeners), antifoam agents, preservatives, buffers, inorganic dispersants, solid carriers or fillers, surfactants, dispersants, emulsifiers, wetters, adjuvants, solubilizers, penetration enhancers, protective colloids, adhesion agents, humectants, repellents, attractants, feeding stimulants, compatibilizers, bactericides, anti-freezing agents, anti-foaming agents, colorants, tackifiers and binders etc., which are usually employed in aqueous formulations active substances.
The amount of auxiliaries will typically not exceed 10% by weight, in particular 5% by weight of the total weight of the formulation.

Such auxiliaries may be incorporated into the aqueous suspension during or after the formation of the microparticles as described herein has been carried out. The amount of additives will generally not exceed 10% by weight, in particular 5% by weight of the total weight of the formulation.

Suitable inorganic dispersants, also termed anticaking agents, for preventing agglutination of the microparticles, are silica (such as, for example Sipernat^{®} 22 from Degussa), alumina, calcium carbonate and the like. In the context of the present invention silica is a preferred inorganic dispersant. The concentration of inorganic dispersants in the final suspension will generally not exceed 2% by weight, based on the total weight of the final suspension, and, if present, it is preferably in the range from 0.01 to 2% by weight, in particular from 0.02 to 1.5% by weight and especially from 0.1 to 1% by weight, based on the total weight of the final formulation.

Suitable thickeners are compounds which affect the flow behavior of the suspension concentrate and may assist in stabilizing the aqueous suspension of the microparticles against caking. Mention may be made, in this connection, for example, of commercial thickeners based on polysaccharides, such as methylcellulose, carboxymethylcellulose, hydroxypropyl cellulose (Klucel^{®} grades), Xanthan Gum (commercially available e.g. as Kelzan^{®} grades from Kelco or Rhodopol^{®} grades from Rhodia), synthetic polymers, such as acrylic acid polymers (Carbopol^{®} grades), polyvinyl alcohol (e.g. Mowiol^{®} and Poval^{®} grades from Kuraray) or polyvinyl pyrrolones, silicic acid or phyllosilicates, such as montmorillonite and bentonites, which may be hydrophobized, (commercially available as Attaclay^{®} grades and Attaflow^{®} grades from BASF SE; or as Veegum^{®} grades and Van Gel^{®} grades from R.T. Vanderbilt). In the context of the present invention, Xanthan Gum is a preferred thickener. The concentration of thickeners in the aqueous suspension will generally not exceed 2% by weight, based on the total weight of the aqueous suspension, and is preferably in the range from 0.01 to 2% by weight, in particular from 0.02 to 1.5% by weight and especially from 0.1 to 1% by weight, based on the total weight of the aqueous suspension or the final formulation, respectively.

Antifoam agents suitable for the compositions according to the invention are, for example, silicone emulsions (such as, for example, Silicone SRE-PFL from Wacker or Rhodorsil^{®} from Bluestar Silicones), polysiloxanes and modified polysiloxanes including polysiloxane blockpolymers such as FoamStar^{®} SI and FoamStar^{®} ST products of BASF SE, long-chain alcohols, fatty acids, organofluorine compounds and mixtures thereof.

Suitable preservatives to prevent microbial spoiling of the compositions of the invention include formaldehyde, alkyl esters of p-hydroxybenzoic acid, sodium benzoate, 2-bromo-2-nitropropane-1,3-diol, o-phenylphenol, thiazolinones, such as benzisothiazolinone, 5-chloro-2-methyl-4-isothiazolinone, pentachlorophenol, 2,4-dichlorobenzyl alcohol and mixtures thereof. Commercially available preservatives that are based on isothiazolinones are for example marketed under the trademarks Proxel^{®} (Arch Chemical), Acticide^{®} MBS (Thor Chemie) and Kathon^{®} MK (Rohm & Haas).
If appropriate, the formulations according to the invention, in particular the aqueous suspensions, may comprise buffers to regulate the pH. Examples of buffers are alkali metal salts of weak inorganic or organic acids such as, for example, phosphoric acid, boric acid, acetic acid, propionic acid, citric acid, fumaric acid, tartaric acid, oxalic acid and succinic acid.

In addition, the compositions according to the invention, in particular the aqueous suspensions, can be formulated with conventional binders, for example aqueous polymer dispersions, water-soluble resins, for example water-soluble alkyd resins, or waxes.

The compositions of the invention may also contain one or more adjuvants. Suitable adjuvants are known to skilled persons and include surfactants, crop oil concentrates, spreader-stickers, wetting agents, and penetrants. In other particular groups of embodiments, the microparticle composition is in the form of solid composition. Such a solid composition contains the microparticles of the invention, optionally one or more surfactants, , and optionally an inert solid carrier material. The solid compositions may e.g. be redispersible powders, water-dispersible granules wettable powders and the like.

Solid carriers include e.g. mineral earths, such as silicas, silica gels, silicates, talc, kaolin, lime stone, lime, chalk, bole, loess, clay, dolomite, diatomaceous earth, calcium sulfate, magnesium sulfate, magnesium oxide, ground synthetic materials, fertilizers such as ammonium sulfate, ammonium phosphate, ammonium nitrate, ureas, and products of vegetable origin, such as cereal meal, tree bark meal, wood meal and nutshell meal, cellulose powders, or other solid carriers.

Suitable **surfactants** are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable **anionic** surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable **nonionic** surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable **cationic** surfactants are quaternary surfactants, for example quaternary ammonium compounds with one or two hydrophobic groups, or salts of long-chain primary amines. Suitable **amphoteric** surfactants are alkylbetains and imidazolines. Suitable **block polymers** are block polymers of the A-B or A-B-A type comprising blocks of polyethylene oxide and polypropylene oxide, or of the A-B-C type comprising alkanol, polyethylene oxide and polypropylene oxide. Suitable **polyelectrolytes** are polyacids or polybases. Examples of polyacids are alkali salts of polyacrylic acid or polyacid comb polymers. Examples of polybases are polyvinylamines or polyethyleneamines.

Suitable **adjuvants** are compounds, which have a neglectable or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable **thickeners** are polysaccharides (e.g. xanthan gum, carboxymethylcellulose), inorganic clays (organically modified or unmodified), polycarboxylates, and silicates.

Suitable **bactericides** are bronopol and isothiazolinone derivatives such as alkylisothiazolinones and benzisothiazolinones.

Suitable **anti-freezing agents** are ethylene glycol, propylene glycol, urea and glycerin.

Suitable **anti-foaming agents** are silicones, long chain alcohols, and salts of fatty acids.

Suitable **colorants** (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable **tackifiers or binders** are polyvinylpyrrolidons, polyvinylacetates, polyvinyl alcohols, polyacrylates, biological or synthetic waxes, and cellulose ethers.

The solid formulations according to the invention may also comprise customary formulation auxiliaries, such as antifoam agents, preservatives, buffers, inorganic dispersants, etc., which are usually employed in solid formulations of active substances. Such auxiliaries may be incorporated into the solid formulation at any conventional stage of their preparation process. The amount of additives will generally not exceed 10% by weight, in particular 5% by weight of the total weight of the solid composition.

Another aspect of the present invention is the use of microparticles or formulations according to the invention or microparticles prepared according to processes of the invention in agrochemical applications (e.g. crop protection, agricultural non-crop applications, seed treatment), pharmaceutical applications, public health, personal care applications (e.g. cosmetic applications), textile applications, human or animal nutrition applications, chemical process applications, adhesives and sealants, paints and coatings, building and construction materials, self-healing materials, tobacco industry, household applications.

In one embodiment microparticles or formulations according to the invention or microparticles prepared according to processes of the invention are used in crop protection.

The inventive microparticles and formulations containing pesticides as active substances are particularly important in the control of a multitude of phytopathogenic fungi, undesired vegetation of insects or nematodes on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (Table grapes and grape juice grape vines); hop; turf; sweet leaf (also called Stevia); natural rubber plants or ornamental and forestry plants, such as flowers, shrubs, broadleaved trees or evergreens, e. g. conifers; and on the plant propagation material, such as seeds, and the crop material of these plants.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays

Especially preferred crops are crops of cereals, corn, soybeans, rice, oilseed rape, cotton, potatoes, peanuts or permanent crops.

In one embodiment microparticles or formulations according to the invention or microparticles prepared according to processes of the invention are used in non-crop applications like home and garden, turf and amenity.

In one embodiment microparticles or formulations according to the invention or microparticles prepared according to processes of the invention are used in seed treatment.
In one embodiment microparticles or formulations according to the invention or microparticles prepared according to processes of the invention are used in pharmaceutical applications.
In one embodiment microparticles or formulations according to the invention or microparticles prepared according to processes of the invention are used in public health applications (e.g. disease control, vector control (e.g. of mosquitos)).
In one embodiment microparticles or formulations according to the invention or microparticles prepared according to processes of the invention are used in personal care applications.
In one embodiment microparticles or formulations according to the invention or microparticles prepared according to processes of the invention are used in cosmetic applications.
In one embodiment microparticles or formulations according to the invention or microparticles prepared according to processes of the invention are used in textile applications.
In one embodiment microparticles or formulations according to the invention or microparticles prepared according to processes of the invention are used in human or animal nutrition applications.
In one embodiment microparticles or formulations according to the invention or microparticles prepared according to processes of the invention are used in chemical process applications.
In one embodiment microparticles or formulations according to the invention or microparticles prepared according to processes of the invention are used in adhesives and sealants.
In one embodiment microparticles or formulations according to the invention or microparticles prepared according to processes of the invention are used in paints and coatings.
In one embodiment microparticles or formulations according to the invention or microparticles prepared according to processes of the invention are used in building and construction materials.
In one embodiment microparticles or formulations according to the invention or microparticles prepared according to processes of the invention are used in self-healing materials.
In one embodiment microparticles or formulations according to the invention or microparticles prepared according to processes of the invention are used in the tobacco industry.
In one embodiment microparticles or formulations according to the invention or microparticles prepared according to processes of the invention are used in household applications.

Another aspect of the present invention are methods for controlling phytopathogenic fungi and/or undesired plant growth and/or undesired attack by insects or mites and/or for regulating the growth of plants, where microparticles according to the invention or prepared according to processes of the invention or formulations according to the invention, in each case containing one or more pesticides as an active substance, are allowed to act on the particular pests, their habitat or the plants to be protected from the particular pest, the soil and/or on undesired plants and/or the useful plants and/or their habitat.

Another aspect of the present invention are seeds containing microparticles of the invention or prepared according to the invention, especially containing one or more pesticides as active substances.

Microparticles according to the invention or prepared according to processes of the invention formulations according to the invention, in each case containing one or more pesticides as an active substance are in one embodiment used for treatment of **treatment of plant propagation materials,** particularly seeds, as part of Suspoemulsions (SE), flowable concentrates (FS), and gels (GF) The formulations in question give, after two-to-tenfold dilution, active substance concentrations of from 0.01 to 60% by weight, preferably from 0.1 to 40% by weight, in the ready-to-use preparations. Application can be carried out before or during sowing. Methods for applying microparticles on plant propagation material, especially seeds, include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.
In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.
When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the microparticles or the formulations comprising them as premix or, if appropriate not until immediately prior to use (tank mix). These agents can be admixed with the compositions according to the invention in a weight ratio of 1:100 to 100:1, preferably 1:10 to 10:1.

The user usually applies the composition according to the invention containing one or more pesticides as active substances in crop protection applications from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

The present invention offers the following advantages:
Microparticles of the invention are environmentally friendly.
Microparticles of the invention only contain naturally occurring polymers in the shell or polymers that are inspired by nature.
Microparticles of the invention do not form any microplastic.
Microparticles of the invention are easily degradable, for example under ambient conditions. Microparticles of the invention contain a shell based on natural materials.
Microparticles of the invention are obtained without any covalent crosslinking. They do not require the use of reactive crosslinking agents. Thus they have a favorable EHS profile and are easy to produce.
Microparticles of the invention allow for a controlled release of active substances. The release profile can be adjusted to the requirements of the application.
Microparticles of the invention can be used in a broad range of applications such as agrochemical applications (e.g. crop protection, agricultural non-crop applications, seed treatment), pharmaceutical applications, public health applications, personal care applications (e.g. cosmetic applications), textile applications, human or animal nutrition applications, chemical process applications, adhesives and sealants, paints and coatings, building and construction materials, self-healing materials, tobacco industry, household applications.
Microparticles of the invention comprising one or more pesticides show a high efficacy for controlling phytopathogenic fungi and/or undesired plant growth and/or undesired attack by insects or mites and/or for regulating the growth of plants.
Microparticles of the invention comprising one or more pheromones show a high efficacy for controlling phytopathogenic fungi and/or undesired plant growth and/or undesired attack by insects or mites and/or for regulating the growth of plants.
Microparticles of the invention are easy and economical to make. They do not require complicated equipment. The can be formed at room temperature and do not requiring cooling during their preparation. They can be prepared in high amounts and processes for their manufacture can be scaled up.
Microparticles and formulations of the invention are storage stable.
Microparticles and formulations of the invention are compatible with a broad range of other active substances and can be formulated with a broad range of other active substances.

### Examples

Particle size Distribution (PSD) was determined by statistic laser scattering using a Malvern Mastersizer 200 according to European norm ISO 13320 EN. The data were treated according to the Mie-Theory by software using a "universal model" provided by Malvern Instruments. Important parameters are the dₙ-values for n = 10, 50 and 90.

Materials used:
Phospholipid A: soybean fluid lecithin (Lecico F200, supplier: Lecico)
Phospholipid B: Asolectin (purified phospholipid product from soybean crop containing lecithin, cephalin, inositol phosphatides & soybean oil, content saturated fatty acids ca. 24 mol%, content monounsaturated fatty acids: ca. 14 mol%, content polyunsaturated fatty acids: 62 mol% (in each case based on the fatty acids, ≥20mol% phosphatidyl choline based (TLC), ca. 25mol% phosphatidylcholine based, supplier: DC Fine Chemicals)
Phospholipid C: soybean fluid lecithin (acid value: max. 35 mg KOH/g, peroxide value: max. 10 meq/kg, viscosity 25 °C: max. 12.5 Pa.s) (Soycithin F60, supplier: Novastell)
Sterol A: Vegapure^{®} FS: betasistosterol (supplier: BASF)
Sterol B: Cholesterol (supplier: Southeast Pharmaceuticals), melting point: 148 °C
Sterol C: General^{®} 98 F: Refined grade natural phytosterol (rape sterols) (supplier: BASF)
Surfactant A ethoxylated sorbitan ester based on oleic acid. polyethylene sorbitol ester, with a calculated molecular weight of 1,310 Daltons, assuming 20 ethylene oxide units, 1 sorbitol, and 1 oleic acid as the primary fatty acid. viscosity 25 °C: 400 - 620 Pa.s; fatty acid composition (as oleic acid): min 58%;
Hydroxyapatite loss on ignition: max 8%; titration (ZnSO₄ 0.1 M): min 90%
Solvent A: aromatic hydrocarbon mixture (Solvesso 200 ND)

### Example 1

First the oil phase composed of 62.42 g cinmethylin, 9.85 g of Phospholipid A and 1.97g Sterol A was prepared in a flask by adding the different components. The flask was sealed and the organic phase was put in water bath and warmed to ca. 50°C under stirring to dissolve completely the components and get a homogenous solution which was cooled down to room temperature afterwards.
In a 500 mL beaker was added 9.85 g Surfactant A and 115.9 g of demineralized water. Under stirring with an ultra-turrax T25 homogenizer at 23,000 rpm, the oil phase was added to the water phase and dispersed at the same speed over 15 min. The temperature did not exceed 30 - 40°C by using an ice bath. After stopping the emulsification, the mixture was allowed to cool down to room temperature and 200 g of dispersion was obtained (d₁₀ = 1.4 µm, d₅₀ = 7.5 µm and d90 = 14.6 µm, pH neat: 4.8).

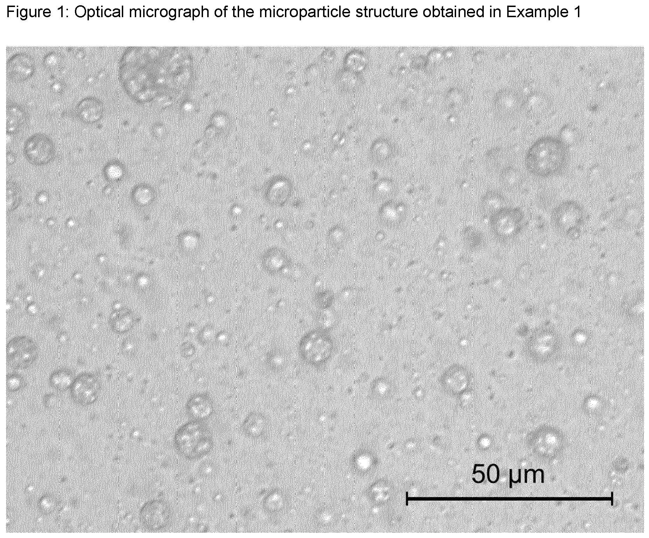

An optical micrograph of the microparticles obtained in Example 1 is shown in Figure 1. The morphology of the microparticles can be seen. The microparticles were spherical with an irregular capsule wall. The size of the capsule was in good agreement with the particle size distribution measured with Malvern Mastersizer 200, no aggregates were observed.

### Example 2

First the oil phase composed of 62.42 g cinmethylin, 9.85 g of Phospholipid A and 1.97g Sterol C was prepared in a flask by adding the different components. The flask was sealed and the organic phase was put in water bath and warmed to ca. 50°C under stirring to dissolve completely the components and get a homogenous solution which was cooled down to room temperature afterwards.
In a 500 mL beaker was added 9.85 g Surfactant A and 115.9 g of demineralized water. Under stirring with an ultra-turrax T25 homogenizer at 23,000 rpm, the oil phase was added to the water phase and dispersed at the same speed over 15 min. The temperature was maintained at 30 - 40°C by using an ice bath. After stopping the emulsification, the mixture was allowed to cool down to room temperature and 200 g of dispersion was obtained (d₁₀ = 1.2 µm, d₅₀ = 6.0 µm and d₉₀ = 12.7 µm, pH neat: 6.2).

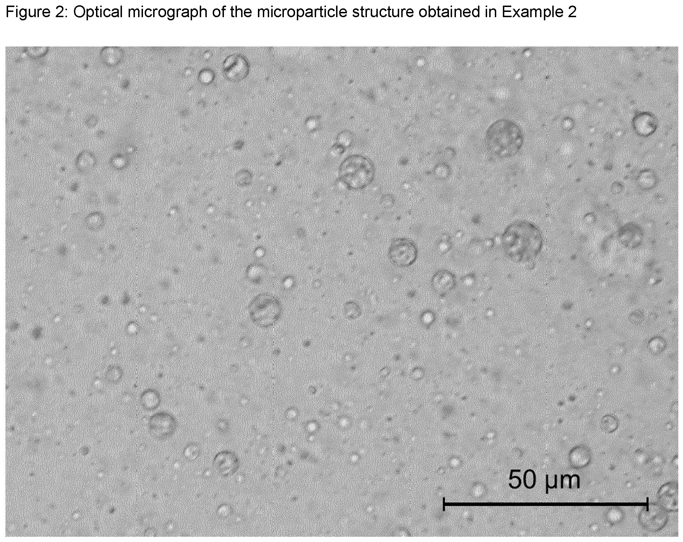

An optical micrograph of the microparticles obtained in Example 2 is shown in Figure 2. The morphology of the microparticles can be seen. The objects were spherical with an irregular capsule wall. The size of the capsule was in good agreement with the particle size distribution measured with Malvern Mastersizer 200, no aggregate was visible.

### Example 3

To a solution of Surfactant A (30.4 g) in demi water (679.8 g, electrical conductivity <2 mS/cm) at 20 - 25 °C, a suspension of Phospholipid B (90.3 g) and Sterol B (18.4 g) in Cinmethylin (359.5 g) was added at the same temperature. The resulting mixture was dispersed by means of IKA Ultra-Turrax T50 homogenizer, operated 8 minutes at 10˙000 rpm. Hydroxyapatite (21.6 g) was then added to the mixture and the resulting suspension was further homogenized 2 minutes at 10˙000 rpm, so to obtain 1200 g of product. The microparticles obtained had an average particle size d₁₀ = 1.5 µm and d₅₀ = 20 µm, the pH neat was 4.8.

### Example 4

To a solution of Tween 20 (3.8 g) in demi water (80.9 g, electrical conductivity <2 mS/cm) at 20 - 25 °C, a suspension of Phospholipid B (16.9 g) in Cinmethylin (45 g) is added at the same temperature. The resulting mixture is dispersed by means of IKA Ultra-Turrax T50 homogenizer, operated 2 minutes at 10˙000 rpm. Hydroxyapatite (3.4 g) is then added to the mixture and the resulting suspension is further homogenized 1 minute at 10˙000 rpm , so to obtain 150 g of product. The microparticles obtained had an average particle size d₁₀ = 1.6 µm and d₅₀ = 20 µm, the pH neat was 5.4.

### Examples 5 and 6

1) A solution of Surfactant A (7.4 g) in demi water (66.3 g, electrical conductivity <2 µS/cm) was prepared by stirring at 20 - 25 °C;
2) A solution of DMTA-P (31.3 g) in Solvent A was prepared by stirring at 20 - 25 °C;
3) To the solution prepared in 2), Sterol C (2.8 g) and either Phospholipid A (11.1 g, for Example 5) or Phospholipid C (11.1 g, for Example 6) were added. The mixture so obtained was stirred at 20 - 25 °C to obtain a homogeneous mixture;
4) The organic mixture prepared in 3) was emulsified into the aqueous solution prepared in 1) with IKA UltraTurrax T50 (10˙000 rpm, 3 mins). Occasional cooling was required in order to keep the temperature at 20 - 25 °C.

### • SAMPLES COMPOSITION

| **Sample ID** | **Example 5** | **Example 6** |
|---|---|---|
| **Capsule material** | Phospholipid A, 11.1 g; | Phospholipid C, 11.1 g; |
| | Sterol C, 2.8 g; | Sterol C, 2.8 g; |
| | Solvent A, 31.3 g | Solvent A, 31.3 g |
| **Active ingredient** | DMTA-P, 31.3 g | DMTA-P, 31.3 g |
| **Protective colloid** | Surfactant A, 7.4 g | Surfactant A, 7.4 g |
| **Solvent** | Water, 66.3 g | Water, 66.3 g |
| **Emulsification conditions** | 10000 rpm; 3 mins; 25 °C | 10000 rpm; 3 mins; 25 °C |
| **Analytical data** | A.i. content 20 %w | A.i. content 20 %w |
| **Observations** | Homogeneous suspension | Homogeneous suspension |
| | | |
| **Malvern practical** | | |
| **laser diffraction** | d₁₀ 0.4 µm | d₁₀ 0.3 µm |
| **particle size analysis:** | d₅₀ 1.2 µm | d₅₀ 0.7 µm |
| **pH neat:** | 4.5 | 4.2 |

## Claims

1. Microparticle, wherein said microparticle contains one or more active substance, said one or more active substance being water immiscible, wherein said one or more active substance is liquid (at 21 °C) or dissolved in a non-aqueous solvent S that is immiscible with water, and
wherein said microparticle contains
i) at least one phospholipid PL,
ii) optionally at least one sterol ST.

2. Microparticle according to claim 1, wherein said microparticle is a microcapsule having a shell and a core or is a microsphere, wherein in phospholipid PL and optionally sterol ST are, in the case of microcapsules, comprised in the shell of such microcapsule.

3. Microparticle according to any of claims 1 to 2, further comprising
iii) an inorganic salt or a mineral, said mineral having a solubility in water of less than 0.01 wt% at 21°C,
wherein said inorganic salt or mineral is present in the form of solid particles having an average particle diameter d50 that is smaller than the particle size of the microparticles wherein said particles of inorganic salt or mineral form a coating on the surface of said microparticle.

4. Microparticle according to any of claims 1 to 3, wherein the inorganic salt or mineral being a phosphate containing inorganic salt or mineral.

5. Microparticle according to any of claims 1 to 4, wherein such microparticles are free from microplastics.

6. Microparticle according to any of claims 1 to 5, wherein said phospholipid PL is selected from asolectin, soy lecithin, sunflower phospholipid.

7. Microparticle according to any of claims 1 to 6, wherein said sterol ST is selected from cholesterol, beta sitosterol, beta sitostanol, stigmasterol, stigmastanol, campesterol, campestanol, ergosterol, avenasterol, brassicasterol, lanosterol, soy sterols, wood sterols, rape sterols.

8. Microparticle according to any of claims 1 to 7, wherein said inorganic salt or a mineral is selected from hydroxy apatite, tricalcium phosphate, calcium hydrogen phosphates.

9. Microparticle according to any of claims 1 to 8, wherein said microparticle further comprises a nonionic surfactant.

10. Microparticle according to any of claims 1 to 9, wherein the mass ratio of component i) to component ii) is from 1:10 to 10:1.

11. Microparticle according to any of claims 1 to 10, wherein the microparticles have an average diameter d50 of 0.1 to 10 µm, preferably 0.5 to 5 µm.

12. Microparticle according to any of claims 1 to 11, wherein the microparticles contain from 1 to 95 wt%, preferably 10 to 90 wt%, 15 to 85 wt% of said one or more active substance.

13. Microparticle according to any of claims 1 to 12, wherein said one or more active substance is selected from pesticides, synergists, plant health agents, repellants, biocides, phase-change materials, pharmaceuticals, cosmetic ingredients (like fragrances, perfumes, vitamins, essential oils, plant extracts), nutrients, food additives (like vegetable oils, marine oils, vitamins, aromas, antioxidants, essential oils, plant extracts), pheromones, catalysts,

14. Process for making microparticles, comprising the following steps:
A) Providing a non-aqueous mixture containing one or more active substance, at least one phospholipid PL, optionally at least one sterol ST and optionally a nonaqueous solvent S that is not miscible with water, wherein phospholipid PL and said sterol ST are at least partially dissolved in said nonaqueous solvent S or said one or more pesticides,
B) Emulsifying the non-aqueous mixture obtained in step A) with water, optionally supported by stirring and/or surfactants,
C) Optionally adding at least one inorganic salt or mineral, preferably phosphate containing salt or mineral, having a solubility in water of less than 0.01 wt% at 21°C.

15. Process according to claims 14, wherein step B) is carried out such that an oil in water emulsion is obtained in step B).

16. Process according to any of claims 14 to 15, wherein said inorganic salt is added in step C) such that the mixture obtained comprises 0.001 to 5 wt%, more preferably 0.002 to 1 wt %, especially preferably 0.005 to 0.1 wt% of said inorganic salt, based on the entire mixture.

17. Formulation comprising Microparticles according to any of claims 1 to 13 or prepared according to claims 14 to 16, wherein said microparticles are present as dispersed particles in an aqueous medium.

18. Formulation according to claim 17, wherein said formulation comprises 1 to 50 wt%, preferably 5 to 45 wt%, more preferably 10 to 40 wt% of said one or more active substances.

19. Use of microparticles according to any of claims 1 to 13 or prepared according to claims 14 to 16 or the formulations according to claims 17 to 18 in agrochemical applications (e.g. crop protection, agricultural non-crop applications, seed treatment), pharmaceutical applications, public health, personal care applications (e.g. cosmetic applications), construction applications, textile applications, human or animal nutrition applications, chemical process applications, adhesives and sealants, paints and coatings, building and construction materials, self-healing materials, tobacco industry, household applications.

20. A method for controlling phytopathogenic fungi and/or undesired plant growth and/or undesired attack by insects or mites and/or for regulating the growth of plants, where Microparticles according to any of claims 1 to 13 or prepared according to claims 14 to 16 or the formulations according to claims 17 to 18 are allowed to act on the particular pests, their habitat or the plants to be protected from the particular pest, the soil and/or on undesired plants and/or the useful plants and/or their habitat.

21. Seeds containing Microparticles according to any of claims 1 to 13 or prepared according to claims 14 to 16.
